# EUROPEAN PATENT SPECIFICATION

(11) **EP 4 412 567 B1**
(45) Date of publication and mention of the grant of the patent: **27.05.2026**
(21) Application number: 22801029.4
(22) Date of filing: 30.09.2022
(51) Int. Cl.: A61F 13/00

(54) **WOUND DRESSING WITH ONE OR MORE COMPOSITE LAYERS**
WUNDVERBAND MIT EINER ODER MEHREREN VERBUNDSCHICHTEN
PANSEMENT COMPRENANT UNE OU PLUSIEURS COUCHES COMPOSITES

(30) Priority: 06.10.2021 GB 202114298
(43) Date of publication of application: 14.08.2024
(60) Divisional application: 26167370.1
(73) Proprietor: T.J.Smith and Nephew,Limited, Hull HU3 2BN (GB)
(72) Inventor: BROWNHILL, Varuni Rachindra, Hull HU3 2BN (GB); DAGGER, Anthony Colin, Hull HU3 2BN (GB); EARL, Michael, Hull HU3 2BN (GB); ELLERINGTON, Mark John, Hull HU3 2BN (GB); FITZGERALD, Daniel James, Hull HU3 2BN (GB); FRY, Nicholas Charlton, Hull HU3 2BN (GB); GOWANS, Philip, Hull HU3 2BN (GB); HAMMOND, Victoria Jody, Hull HU3 2BN (GB); KOTECHA, Bindiya, Hull HU3 2BN (GB); LECOMTE, Helene Anne, Hull HU3 2BN (GB); MIDDLETON, Natasha Rose, Hull HU3 2BN (GB)
(74) Representative: Appleyard Lees IP LLP
(86) International application number: PCT/EP2022/077401
(87) International publication number: WO 2023/057355

(56) References cited:
- WO-A1-2008/116497
- WO-A1-2010/048724
- WO-A1-2016/079538
- WO-A1-2021/059124
- US-A1- 2006 034 816
- US-A1- 2009 214 624

## Description

### BACKGROUND

### Technical Field

Disclosed herein are materials, devices, methods, and systems, such as therapeutic compositions, wound care materials, their uses, and methods of treatment therewith. In some examples, the materials, devices, and systems described herein comprise a wound dressing configured for nitric oxide (NO) delivery and/or the delivery of other actives.

### Description of the Related Art

Nitric oxide (NO) is a well-known molecule with multiple biological functions. For example, nitric oxide influences blood vessel vasodilation, stimulates angiogenesis, influences the host immune response, and demonstrates potent, broad spectrum antimicrobial activity and anti-biofilm activity. Due to these multiple roles, NO demonstrates a potent effect on tissue and increased amounts of NO may support the acceleration of healing in wounds, particularly chronic wounds.

Additionally, diabetic patients often have lower levels of nitric oxide as compared to healthy patients, and diminished supply of nitric oxide in diabetic patients is a compounding factor in a healing chronic ulcer. Diminished supply of nitric oxide may lead to vascular damage, such as endothelial dysfunction and vascular inflammation. Vascular damage may also lead to decreased blood flow to the extremities, thereby potentially causing the diabetic patient to be more likely to develop neuropathy and non-healing ulcers, and to be at a greater risk for lower limb amputation.

Consequently, there is a need for improved mechanisms of delivering an effective dose of nitric oxide to a wound. Under normal conditions, nitric oxide (NO), a free radical, is short-lived and converted to a more stable chemical species within seconds of production. Thus, for example, if gaseous nitric oxide contacts air, the gaseous nitric oxide will be rapidly oxidized to generate nitrogen dioxide (NO₂). Accordingly, it may be difficult to maintain high concentrations of nitric oxide within a wound dressing or other similar structure for a prolonged period of time. Therefore, a device or a wound dressing having one or more layers containing more stable compositions may effectively generate nitric oxide over time upon activation, for the stable and sustained delivery of nitric oxide to biological tissues. Of particular interest are mechanisms of delivering nitric oxide in combination with use of a wound dressing, particularly a negative pressure wound dressing and/or while undergoing negative pressure wound therapy and/or other appropriate therapies.

US2009214624A1 relates to topical nitric oxide delivery systems, and to using the same for mitigating or remediating various disease states. WO2010048724A1 relates to the use of nitric-oxide-releasing anti-septic agents in wound dressings. WO2008116497A1 relates to a topical dermal delivery device, such as a patch, for topical delivery of nitric oxide (NO) to treatment sites of mammals. WO2021059124A1 relates to a wound dressing which includes a patient interface layer, a wicking layer, an absorbent layer, and a cover layer. WO2016079538A1 relates to skin dressings that are useful in the treatment of conditions associated with tissue ischaemia and skin lesions such as diabetic foot ulcers, burns and surgical wounds. US2006034816A1 relates to a skin dressing, particularly a wound dressing, comprising oxidoreductase enzyme and, optionally, peroxidase enzyme, wherein the enzyme(s) are present in hydrated condition, e.g. being present in one or more hydrated hydrogels.

### SUMMARY

Embodiments of the present disclosure relate to materials, devices, methods, and systems for wound treatment. Some disclosed embodiments relate to materials, devices, methods, and systems for delivering nitric oxide to a wound. It will be understood by one of skill in the art that application of the materials, devices, methods, and systems described herein are not limited to a particular tissue or a particular injury.

A wound dressing for treating a wound is provided as set out in accompanying claim 1.

Also disclosed is a wound dressing for treating a wound comprising a cover layer configured to form a seal around a wound, an activator layer, a dry nitric oxide source layer, the dry nitric oxide source layer free or relatively free of liquid, and an acquisition distribution layer. The wound dressing may further comprise a masking layer, the masking layer configured to at least partially limit visualization of the wound. The dry nitric oxide source layer may comprise a nitrite salt. The nitrite salt may comprise sodium nitrite. The activator layer may be positioned above the nitric oxide source layer. In some embodiments, the nitric oxide source layer may be positioned above the activator layer. The acquisition distribution layer may be positioned between the activator layer and the dry nitric oxide source layer. The activator layer may comprise a hydrogel or a xerogel. The wound dressing may comprise a second dry nitric oxide source layer. The wound dressing may be configured to generate nitric oxide when the wound dressing is placed over a wound. In embodiments, the wound dressing may be configured to not generate nitric oxide prior to placement over a wound.

The wound dressing for treating a wound according to claim 1 comprises:
a cover layer configured to form a seal around a wound;
a composite acquisition distribution layer, the composite acquisition distribution layer comprising an activator gel; and
a nitrite source layer.

The activator gel may include acidic groups or moieties. The wound dressing may be configured to generate nitric oxide when the wound dressing interacts with fluid. The fluid may comprise wound exudate. In some embodiments, the composite acquisition distribution layer comprises a plurality of perforations. The perforations may comprise an open area of about 25 to 75% of the area of the acquisition distribution layer. The open area may be about 50%. The composite layer is formed from an activator gel cast on an acquisition distribution layer.

A wound dressing for treating a wound is provided as set out in accompanying claim 8. The wound dressing for treating a wound comprises:
a cover layer configured to form a seal around a wound;
a composite absorbent layer, the composite absorbent layer comprising an activator gel; and
a nitrite source layer.

The composite absorbent layer further comprises cellulose fibers, gelling fibers, or the activator gel is cast on a foam layer. The composite absorbent layer may comprise ethyl sulphonate fibers. In certain embodiments, the composite absorbent layer may comprise superabsorbent and/or the gelling fiber may comprise superabsorbent. The activator gel may be in the form of an array of rounded shapes and/or the activator gel may be in the form of a plurality of discs embedded in the foam. The activator gel may include acidic groups or moieties.

Alternative or additional embodiments described herein provide a composition comprising one or more of the features of the foregoing description or of any description elsewhere herein.

Alternative or additional embodiments described herein provide a wound contact layer comprising one or more of the features of the foregoing description or of any description elsewhere herein.

Alternative or additional embodiments described herein provide a wound dressing comprising one or more of the features of the foregoing description or of any description elsewhere herein.

Alternative or additional embodiments described herein provide a wound treatment system comprising one or more of the features of the foregoing description or of any description elsewhere herein.

Alternative or additional embodiments described herein provide a method of treating a wound comprising one or more of the features of the foregoing description or of any description elsewhere herein.

### BRIEF DESCRIPTION OF THE DRAWINGS

FIG. 1 is a schematic diagram of an example of a negative pressure wound therapy system;
FIG. 2A illustrates an embodiment of a negative pressure wound treatment system employing a pump, a flexible fluidic connector and a wound dressing capable of absorbing and storing wound exudate;
FIG. 2B illustrates an embodiment of a negative pressure wound treatment system employing a flexible fluidic connector and a wound dressing capable of absorbing and storing wound exudate;
FIG. 2C illustrates a cross section of an embodiment of a fluidic connector connected to a wound dressing;
FIG. 2D illustrates a cross-section of an embodiment of a wound dressing;
FIGS. 3A-3D illustrate embodiments of wound dressings capable of absorbing and storing wound exudate to be used without negative pressure;
FIG. 3E illustrates a cross section of an embodiment of a wound dressing capable of absorbing and storing wound exudate to be used without negative pressure;
FIG. 4 is an exploded view of an embodiment of a wound dressing which can generate nitric oxide;
FIG. 5 is a cross sectional view of the wound dressing of FIG. 4;
FIG. 6 illustrates an example of a chemiluminescence experimental protocol equipment setup;
FIGS. 7A-B illustrates a negative pressure and nitric oxide delivery experiment;
FIG. 8A depicts an example of chemiluminescence experimental results for a sodium nitrate mesh;
FIG. 8B depicts an example of chemiluminescence experimental results for a full dressing design with a pull-out tab and self-sealing borders;
FIG. 8C depicts an example of chemiluminescence experimental results for a dressing containing a degradable film;
FIG. 9 depicts an example of a graph displaying peak NO and NO₂ outputs for acrylic adhesive containing hydrogels;
FIGS. 10A-D depict examples of chemiluminescence experimental results for a nitric oxide producing dressing;
FIGS. 11A-D depicts embodiments of a wound dressing configured to generate nitric oxide;
FIG. 12 depicts an exploded view of an embodiment of a wound dressing configured to generate nitric oxide;
FIG. 13 depicts a side view of an embodiment of a wound dressing configured to generate nitric oxide;
FIG. 14 depicts an exploded view of an embodiment of a wound dressing with multiple acquisition distribution layers, the wound dressing configured to generate nitric oxide;
FIG. 15 depicts a side view of an embodiment of a wound dressing with multiple acquisition distribution layers, the wound dressing configured to generate nitric oxide;
FIG. 16 depicts an embodiment of a process for implanting or adhering a material layer;
FIG. 17 depicts an embodiment of a process for implanting or adhering a material layer;
FIGS. 18A-B depicts an embodiment of a wound dressing with a composite acquisition distribution layer;
FIGS. 19A-B show embodiments of fluid handling tests on wound dressings with a composite layer;
FIG. 20 depicts a side view of an embodiment of a wound dressing including a composite absorbent layer;
FIGS. 21A-E depict embodiments of wound dressings including a composite absorbent layer;
FIG. 22 depicts an embodiment of a wound dressing including a composite absorbent layer;
FIGS. 23A-E depicts embodiments of wound dressings with perforated composite acquisition distribution layers;
FIG. 24 depicts an embodiment of a wound dressing with a composite layer;
FIG. 25 depicts an embodiment of a wound dressing with a spring composite layer;
FIG. 26 depicts an embodiment of a wound dressing with alternating composite and gel layers;
FIG. 27 depicts an embodiment of a wound dressing with an extended lower layer;
FIG. 28 depicts an embodiment of a composite layer;
FIG. 29 depicts an embodiment of a composite layer with gel surrounded by absorbent;
FIG. 30 depicts an embodiment of a composite wound dressing with an absorbent nitrite providing layer;
FIG. 31 depicts an embodiment of a composite wound dressing with a crumpled nitrite providing layer.

### DETAILED DESCRIPTION

### Overview

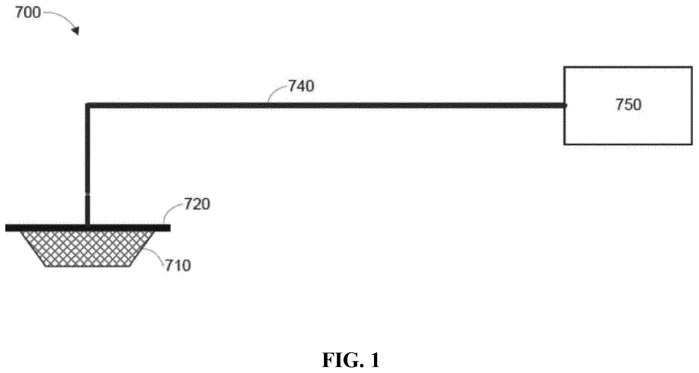

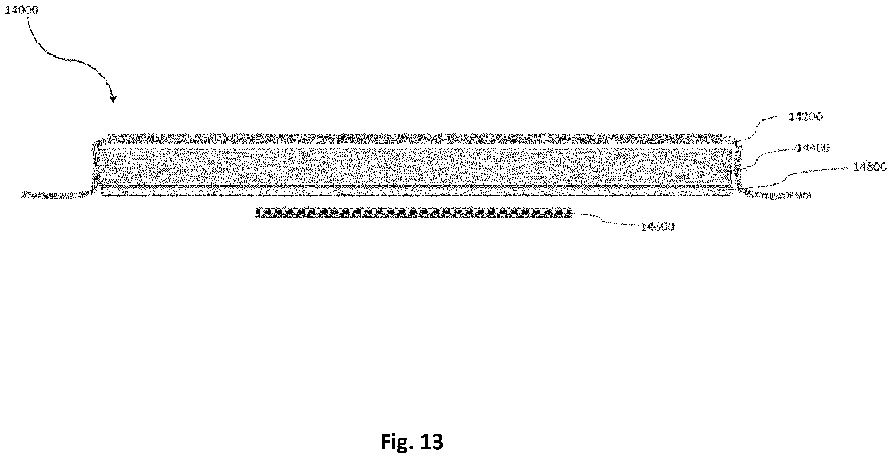

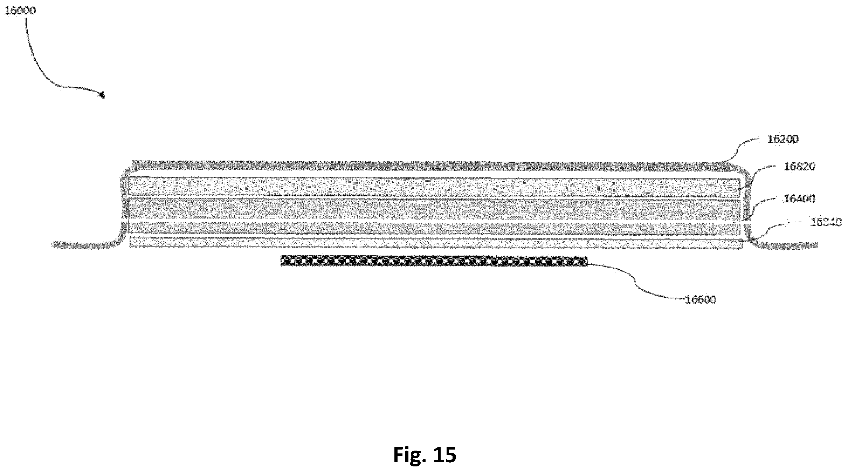

Embodiments described herein relate to materials, apparatuses, methods, and systems that incorporate, or comprise, or utilize one or more compositions and/or materials that effectively generate gases (e.g. nitric oxide) over time upon activation. Embodiments herein may be directed toward a device and/or a wound dressing having one or more layers containing compositions and/or materials that effectively generate nitric oxide over time upon activation. For example, one or more nitric oxide generating layers may include a nitrite delivery layer which contains nitrite salts and can release nitrite ions, such that the nitrite ions can generate nitric oxide upon reaction with acids. In some embodiments, the one or more nitric oxide generating layers can further include an acidic-group-providing layer in addition to the nitrite delivery layer. The one or more nitric oxide generating layers may be utilized as a stand-alone component for separately positioning at a wound site, or may be incorporated into any number of multi-layer wound dressings and wound treatment apparatuses, such as described herein below with respect to Figures 1 through 31. Embodiments of the present disclosure are generally applicable to use under ambient conditions, in negative pressure or reduced pressure therapy systems, or in compression therapy systems.

Some of the preferred embodiments described herein incorporate, or comprise, or utilize one or more nitric oxide generating layers. Such one or more nitric oxide generating layers may possess one or more of the following functional features: inflammation-related activities, blood flow-related activities, antimicrobial, anti-planktonic and anti-biofilm activities, ease of application or/and removal as one piece, cuttability/tearability, conformability to the three-dimensional contour of a wound surface, durability to wear, compatibility with negative pressure wound therapy or/and compression wound therapy, exudate management, capability of facilitating autolytic debridement of wounds, capability of promoting wound healing, and self-indication of compositional or functional changes. The antimicrobial activities, such as *in vitro* antimicrobial activities, can include one or more of the following: broad-spectrum antimicrobial activity, anti-biofilm activity, rapid speed of kill against microorganisms, sustained kill against microorganisms; and the microorganisms can include one or more of the following: Gram-negative bacteria, Gram-positive bacteria, fungi, yeasts, viruses, algae, archaea and protozoa.

Also disclosed is a wound treatment system. Such a wound treatment system may comprise nitric oxide generating layers, configured to be sized for positioning over a wound and/or the periwound area. One of skill in the art will understand that when an apparatus/dressing/layer is described as being placed on or over a wound, such an apparatus/dressing/layer may extend over and treat the periwound area. In some instances, stimulation of the periwound area and/or the wound edge may play a role in initiating the wound healing process, and the wound healing process can be activated through the delivery of nitric oxide to the periwound area and/or the wound edge. The delivery of nitric oxide to the periwound area and/or the wound edge may target, for example epithelial cell activity to promote migration of epithelial tongue; vasodilation of the microcirculation in the skin surrounding the wound to promote profusion by providing oxygen and nutrients; and neo-angiogenesis to promote granulation tissue formation. The wound treatment systems described herein may further comprise a secondary wound dressing configured to be separately positioned over the nitric oxide generating layers. The nitric oxide generating layers may have an adhesive adhered to the lower surface; and the adhesive can be configured such that the nitric oxide generating layers may be placed in proximity to the wound. The secondary wound dressing, if used, may adhere to skin surrounding the wound and may have the same size or may be larger than the nitric oxide generating layers, such that the nitric oxide generating layers will touch or be placed in proximity to the wound and/or the periwound area. The secondary wound dressing can be alternatively or additionally configured to form a seal to skin surrounding the wound so that the nitric oxide generating layers will touch or be placed in proximity to the wound. The wound treatment system may further comprise a source of negative pressure configured to supply negative pressure through the secondary wound dressing and through the wound contact layer to the wound.

Certain preferred embodiments described herein provide a multi-layered wound dressing, such as described herein the specification with respect to FIGS. 1 through 31. Such a multi-layered wound dressing may incorporate the one or more nitric oxide generating layers as component layers thereof or, alternatively, may comprise a composite or laminate including the one or more nitric oxide generating layers as part of one of the component layers thereof. The multi-layered wound dressing may comprise: nitric oxide generating layers as described above or described elsewhere herein; a transmission layer and/or absorbent layer over/under the one or more nitric oxide generating layers; a wound contact layer under the one or more nitric oxide generating layers; and a cover layer over the transmission layer and/or absorbent layer. The wound dressing may further comprise a negative pressure port positioned on or above the cover layer. The one or more nitric oxide generating layers may have a perimeter shape that is substantially the same as a perimeter shape of the cover layer. Alternatively, the one or more nitric oxide generating layers may have a perimeter shape that is smaller than a perimeter shape of the cover layer.

One of skill in the art will understand that nitric oxide generating compositions, such as any disclosed herein this "Overview" section or elsewhere in the specification, may be loaded within the one or more nitric oxide generating layers in any suitable form, such as via adsorption, absorption, chemical and/or physical attachment entanglement, and/or via powder form. One of skill in the art will further understand that reactive compositions, such as any disclosed herein this section or elsewhere in the specification may be incorporated into any suitable absorbent layer disclosed herein this section or elsewhere in the specification by any suitable means, and/or any suitable transmission layer disclosed herein this section or elsewhere in the specification, and/or any foam layer disclosed herein this section or elsewhere in the specification.

In certain embodiments, the wound treatment systems and multi-layered wound dressings disclosed above or disclosed elsewhere herein the specification may incorporate or comprise nitric oxide generating layers. As described herein this section or elsewhere in the specification, particularly below, the nitric oxide generating layers may be configured to be activated to release nitric oxide. At least a portion of the released nitric oxide may be released, for example by diffusion. To facilitate release and diffusion of nitric oxide, the nitric oxide generating layers may be placed proximate to the wound.

Also disclosed herein is a method to treat a wound, intact tissue, or other suitable location. Such a method may include placing nitric oxide generating layers, either separately or by placing a multi-layered wound dressing having nitric oxide generating layers, over the wound. The method may comprise adhering the separate nitric oxide generating layers and/or the multi-layer wound dressing having nitric oxide generating layers to healthy skin around the wound. Such a method may further comprise one or more of the following steps: A further wound dressing can be placed over the separate nitric oxide generating layers or multi-layered wound dressing having the nitric oxide generating layers that is placed over the wound. Wound exudate, or any moist or aqueous medium other than wound exudate, may be provided to reach and/or touch the nitric oxide generating layers. Wound exudate, or any moist or aqueous medium other than wound exudate may be diffused or wicked into the wound dressing incorporating the nitric oxide generating layers or into a wound dressing provided over the nitric oxide generating layers. Negative pressure may be applied to the separate nitric oxide generating layers or multi-layered wound dressing having the nitric oxide generating layers, such that wound exudate is suctioned into the nitric oxide generating layers directly, or into the wound dressing incorporating the nitric oxide generating layers, or into a wound dressing provided over the nitric oxide generating layers.

One of skill in the art will understand that wound dressings, devices and systems disclosed herein this "Overview" section or elsewhere in the specification may include one or more layers, compositions, materials or components that generate gases other than nitric oxide in addition to or in place of the nitric oxide generating layers, compositions or materials. For example, a wound dressing or a device can include one or more layers that effectively generate vasodilatory agents, such as carbon monoxide or hydrogen sulfide, over time upon activation.

One of skill in the art will further understand that carbon monoxide and/or hydrogen sulfide may be used in place of a nitric oxide delivery element (such as a layer) or in combination with a nitric oxide delivery element (such as a layer) where suitable. Further details regarding generation and delivery of carbon monoxide and/or hydrogen sulfide may be found in chapter six of the text Inorganic and Organometallic Transition Metal Complexes with Biological Molecules and Living Cells, ISBN 978-0-12-803814-7, which is hereby incorporated by reference. For example, hydrogen sulfide may be generated from elements/layers that contain cleavable/releasable hydrogen sulfide, diallyl thiosulfinate, GYY4137, S-Mesalamine ATB-429, S-Naproxen ATB-346, S-Diclofenac ATB-337/ACS-15. For example, carbon monoxide may be generated from elements/layers that provide of complexes of carbon monoxide bound to suitable metals such as chromium, molybdenum, tungsten, manganese, rhenium, iron, ruthenium, cobalt, rhodium, and iridium. Such complexes may be enzymatically triggered to release carbon monoxide, photo-cleavable, and/or responsive to interaction with a suitable ligand to induce release of carbon monoxide.

### Method of Treating a Wound

Disclosed herein is a method of treating a wound, intact tissue, or other suitable location. Such a method may include placing one or more nitric oxide generating layers, either separately or by placing a multi-layered wound dressing having one or more nitric oxide generating layers over the wound. The method may comprise adhering the separate one or more nitric oxide generating layers and/or the multi-layer wound dressing having one or more nitric oxide generating layers to healthy skin around the wound, such as the periwound area. The method may further comprise one or more of the following steps: A further wound dressing can be placed over the separate one or more nitric oxide generating layers or multi-layered wound dressing having the one or more nitric oxide generating layers that is placed over the wound. Wound exudate, or any moist or aqueous medium other than wound exudate, may be provided to reach and/or touch the one or more nitric oxide generating layers. Wound exudate, or any moist or aqueous medium other than wound exudate may be diffused or wicked into the wound dressing incorporating the one or more nitric oxide generating layers or into a wound dressing provided over the one or more nitric oxide generating layers. Negative pressure may be applied to the separate one or more nitric oxide generating layers or multi-layered wound dressing having the one or more nitric oxide generating layers, as described in the following "Negative Pressure Wound Therapy (NPWT) Systems" section or described elsewhere herein the specification, such that wound exudate is suctioned into the one or more nitric oxide generating layers directly, or into the wound dressing incorporating the one or more nitric oxide generating layers, or into a wound dressing provided over the one or more nitric oxide generating layers.

The method of treating a wound, intact tissue, or other suitable location as described above or described elsewhere herein may further comprise delivering negative pressure through the wound contact layer to the wound, as described in the following "Negative Pressure Wound Therapy (NPWT) Systems" section or described elsewhere herein the specification. The wound contact layer may substantially maintain the negative pressure delivered for at least about 24 hours, or for at least about 48 hours, or for at least about 72 hours. Alternatively, the method of treating a wound, intact tissue, or other suitable location may comprise applying compression (positive) pressure through the wound contact layer to the wound. Alternatively, the method may comprise altering ambient pressure, negative pressure and compression pressure in a programmable manner through the wound contact layer to the wound.

The method of treating a wound, intact tissue, or other suitable location may comprise using the wound contact layer, or the wound treatment system or wound dressing that comprises the wound contact layer, under ambient conditions not in connection with a negative pressure wound therapy system as described above, or described elsewhere herein.

The method of treating a wound, intact tissue, or other suitable location may reduce the wound bioburden, for example, at least *in vitro,* by reducing the numbers (CFU/sample) of viable microorganisms within the first 4 hours after the application wound contact layer. In some examples, the numbers of viable microorganisms may be reduced by four log or more, 48 to 72 hours after positioning the wound dressing in contact with the microorganisms.

### Negative Pressure Wound Therapy (NPWT) Systems

It will be understood that embodiments of the present disclosure are generally applicable to, but not limited to, use in topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (e.g., - 40 mmHg is less than -60 mmHg). Negative pressure that is "more" or "greater" than -X mmHg corresponds to pressure that is further from atmospheric pressure (e.g., -80 mmHg is more than - 60 mmHg). In some embodiments, local ambient atmospheric pressure is used as a reference point, and such local atmospheric pressure may not necessarily be, for example, 760 mmHg.

The negative pressure range for some embodiments of the present disclosure can be approximately -80 mmHg, or between about -20 mmHg and -200 mmHg. Note that these pressures are relative to normal ambient atmospheric pressure, which can be 760 mmHg. Thus, - 200 mmHg would be about 560 mmHg in practical terms. In some embodiments, the pressure range can be between about -40 mmHg and -150 mmHg. Alternatively, a pressure range of up to -75 mmHg, up to -80 mmHg or over -80 mmHg can be used. Also in other embodiments a pressure range of below -75 mmHg can be used. Alternatively, a pressure range of over approximately - 100 mmHg, or even -150 mmHg, can be supplied by the negative pressure apparatus.

In some embodiments of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some embodiments, negative pressure may be varied over time for example using a sinusoidal wave, square wave, or in synchronization with one or more patient physiological indices (e.g., heartbeat). Examples of such applications where additional disclosure relating to the preceding may be found include U.S. Patent No. 8,235,955, titled "Wound treatment apparatus and method," issued on August 7, 2012; and U.S. Patent No. 7,753,894, titled "Wound cleansing apparatus with stress," issued July 13, 2010.

Embodiments of the wound dressings, wound dressing components, wound treatment apparatuses and methods described herein may also be used in combination or in addition to those described in International Application No. PCT/IB2013/001469, filed May 22, 2013, published as WO 2013/175306 A2 on November 28, 2013, titled "APPARATUSES AND METHODS FOR NEGATIVE PRESSURE WOUND THERAPY," International Application No. PCT/IB2013/002060, filed on July 31, 2013, published as WO2014/020440, entitled "WOUND DRESSING". Embodiments of the wound dressings, wound treatment apparatuses and methods described herein may also be used in combination or in addition to those described in US Patent No. 9,061,095, titled "WOUND DRESSING AND METHOD OF USE," issued on June 23, 2015; and U.S. Application Publication No. 2016/0339158, titled "FLUIDIC CONNECTOR FOR NEGATIVE PRESSURE WOUND THERAPY," published on November 24, 2016 including further details relating to embodiments of wound dressings, the wound dressing components and principles, and the materials used for the wound dressings.

Additionally, some embodiments related to TNP wound treatment comprising a wound dressing in combination with a pump or associated electronics described herein may also be used in combination or in addition to those described in International Publication No. WO 2016/174048 A1, entitled "REDUCED PRESSURE APPARATUSES", published on November 3, 2016. In some of these embodiments, the pump or associate electronic components may be integrated into the wound dressing to provide a single article to be applied to the wound.

### Multi-Layered Wound Dressings for NPWT

Figure 1 illustrates an example of a negative pressure wound therapy system 700. The system includes a wound cavity 710 covered by a wound dressing 720, which can be a dressing according to any of the examples described herein. The dressing 720 can be positioned on, inside, over, or around the wound cavity 710 and further seal the wound cavity so that negative pressure can be maintained in the wound cavity. For example, a film layer of the wound dressing 720 can provide substantially fluid impermeable seal over the wound cavity 710. In some embodiments, a wound filler, such as a layer of foam or gauze, may be utilized to pack the wound. The wound filler may include one or more nitric oxide generating layers (e.g. a nitrite delivery layer, an acidic-group providing layer) as described herein this section or elsewhere in the specification. For example, in a traditional negative pressure wound therapy system utilizing foam or gauze, such as the Smith + Nephew RENASYS Negative Pressure Wound Therapy System utilizing foam (RENASYS-F) or gauze (RENASYS-G), the foam or gauze may be supplemented with nitric oxide generating layers as described above. When supplementing a foam or gauze layer or other wound packing material, the one or more nitric oxide generating layers may either be separately inserted into the wound or may be pre-attached with the wound packing material for insertion into the wound.

A single or multi lumen tube or conduit 740 connects the wound dressing 720 with a negative pressure device 750 configured to supply reduced pressure. The negative pressure device 750 includes a negative pressure source. The negative pressure device 750 can be a canisterless device (meaning that exudate is collected in the wound dressing and/or is transferred via the tube 740 for collection to another location). In some embodiments, the negative pressure device 750 can be configured to include or support a canister. Additionally, in any of the embodiments disclosed herein, the negative pressure device 750 can be fully or partially embedded in, mounted to, or supported by the wound dressing 720.

The conduit 740 can be any suitable article configured to provide at least a substantially sealed fluid flow path or pathway between the negative pressure device 750 and the wound cavity 710 so as to supply reduced pressure to the wound cavity. The conduit 740 can be formed from polyurethane, PVC, nylon, polyethylene, silicone, or any other suitable rigid or flexible material. In some embodiments, the wound dressing 720 can have a port configured to receive an end of the conduit 740. For example, a port can include a hole in the film layer. In some embodiments, the conduit 740 can otherwise pass through and/or under a film layer of the wound dressing 720 to supply reduced pressure to the wound cavity 710 so as to maintain a desired level of reduced pressure in the wound cavity. In some embodiments, at least a part of the conduit 740 is integral with or attached to the wound dressing 720.

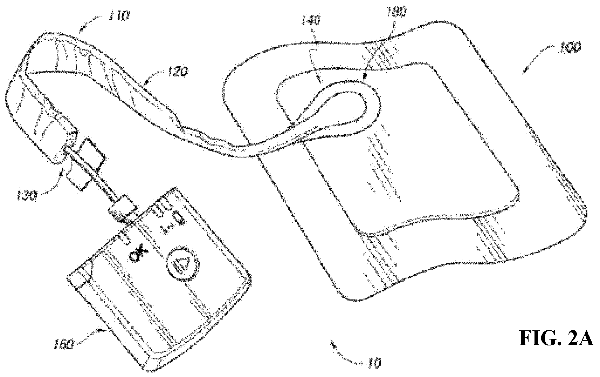

Figure 2A illustrates an embodiment of a negative pressure wound treatment system 10 employing a wound dressing 100 in conjunction with a fluidic connector 110. Additional examples related to negative pressure wound treatment comprising a wound dressing in combination with a pump as described herein may also be used in combination or in addition to those described in US Patent No. 9,061,095. Here, the fluidic connector 110 may comprise an elongate conduit, more preferably a bridge 120 having a proximal end 130 and a distal end 140, and an applicator 180 at the distal end 140 of the bridge 120. The system 10 may include a source of negative pressure such as a pump or negative pressure unit 150 capable of supplying negative pressure. The pump may comprise a canister or other container for the storage of wound exudates and other fluids that may be removed from the wound. A canister or container may also be provided separate from the pump. In some embodiments, the pump 150 can be a canisterless pump such as the PICO^{™} pump, as sold by Smith + Nephew. The pump 150 may be connected to the bridge 120 via a tube, or the pump 150 may be connected directly to the bridge 120. In use, the dressing 100 is placed over a suitably-prepared wound, which may in some cases be filled with a wound packing material such as foam or gauze as described above. The applicator 180 of the fluidic connector 110 has a sealing surface that is placed over an aperture in the dressing 100 and is sealed to the top surface of the dressing 100. Either before, during, or after connection of the fluidic connector 110 to the dressing 100, the pump 150 is connected via the tube to a coupling of the tube, or is connected directly to the bridge 120. The pump is then activated, thereby supplying negative pressure to the wound. Application of negative pressure may be applied until a desired level of healing of the wound is achieved.

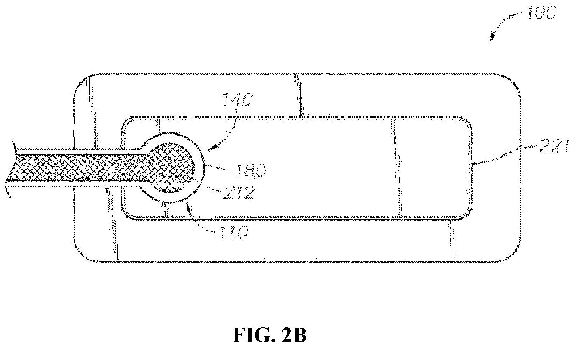

As shown in Figure 2B, the fluidic connector 110 preferably comprises an enlarged distal end, or head 140 that is in fluidic communication with the dressing 100 as will be described in further detail below. In one embodiment, the enlarged distal end has a round or circular shape. The head 140 is illustrated here as being positioned near an edge of the dressing 100, but may also be positioned at any location on the dressing. For example, some embodiments may provide for a centrally or off-centered location not on or near an edge or corner of the dressing 100. In some embodiments, the dressing 10 may comprise two or more fluidic connectors 110, each comprising one or more heads 140, in fluidic communication therewith. In a preferred embodiment, the head 140 may measure 30mm along its widest edge. The head 140 forms at least in part the applicator 180, described above, that is configured to seal against a top surface of the wound dressing.

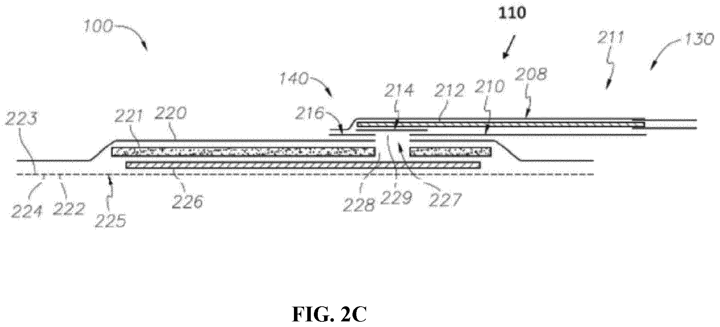

Figure 2C illustrates a cross-section through a wound dressing 100 similar to the wound dressing 10 as described in International Patent Publication WO2013175306 A2 along with fluidic connector 110. The wound dressing 100, which can alternatively be any wound dressing embodiment disclosed herein or any combination of features of any number of wound dressing embodiments disclosed herein, can be located over a wound site to be treated. The dressing 100 may be placed as to form a sealed cavity over the wound site. In a preferred embodiment, the dressing 100 comprises a top or cover layer, or backing layer 220 attached to an optional wound contact layer 222, both of which are described in greater detail below. These two layers 220, 222 are preferably joined or sealed together so as to define an interior space or chamber. This interior space or chamber may comprise additional structures that may be adapted to distribute or transmit negative pressure, store wound exudate and other fluids removed from the wound, and other functions which will be explained in greater detail below. Examples of such structures, described below, include a transmission layer 226 and an absorbent layer 221.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound.

As illustrated in Figure 2C, the wound contact layer 222 can be a polyurethane layer or polyethylene layer or other flexible layer which is perforated, for example via a hot pin process, laser ablation process, ultrasound process or in some other way or otherwise made permeable to liquid and gas. The wound contact layer 222 has a lower surface 224 and an upper surface 223. The perforations 225 preferably comprise through holes in the wound contact layer 222 which enable fluid to flow through the layer 222. The wound contact layer 222 helps prevent tissue ingrowth into the other material of the wound dressing. Preferably, the perforations are small enough to meet this requirement while still allowing fluid to flow therethrough. For example, perforations formed as slits or holes having a size ranging from 0.025 mm to 1.2 mm are considered small enough to help prevent tissue ingrowth into the wound dressing while allowing wound exudate to flow into the dressing. In some configurations, the wound contact layer 222 may help maintain the integrity of the entire dressing 100 while also creating an air tight seal around the absorbent pad in order to maintain negative pressure at the wound.

Some embodiments of the wound contact layer 222 may also act as a carrier for an optional lower and upper adhesive layer (not shown). For example, a lower pressure sensitive adhesive may be provided on the lower surface 224 of the wound dressing 100 whilst an upper pressure sensitive adhesive layer may be provided on the upper surface 223 of the wound contact layer. The pressure sensitive adhesive, which may be a silicone, hot melt, hydrocolloid or acrylic based adhesive or other such adhesives, may be formed on both sides or optionally on a selected one or none of the sides of the wound contact layer. When a lower pressure sensitive adhesive layer is utilized may be helpful to adhere the wound dressing 100 to the skin around a wound site. In some embodiments, the wound contact layer may comprise perforated polyurethane film. The lower surface of the film may be provided with a silicone pressure sensitive adhesive and the upper surface may be provided with an acrylic pressure sensitive adhesive, which may help the dressing maintain its integrity. In some embodiments, a polyurethane film layer may be provided with an adhesive layer on both its upper surface and lower surface, and all three layers may be perforated together.

A transmission layer 226 can be located above the wound contact layer 222. In some embodiments, the transmission layer can be a porous material. As used herein the transmission layer can be referred to as a spacer layer and the terms can be used interchangeably to refer to the same component described herein. This transmission layer 226 allows transmission of fluid including liquid and gas away from a wound site into upper layers of the wound dressing. In particular, the transmission layer 226 preferably ensures that an open-air channel can be maintained to communicate negative pressure over the wound area even when the absorbent layer has absorbed substantial amounts of exudates. The layer 226 should preferably remain open under the typical pressures that will be applied during negative pressure wound therapy as described above, so that the whole wound site sees an equalized negative pressure. The layer 226 may be formed of a material having a three-dimensional structure. For example, a knitted or woven spacer fabric (for example Baltex 7970 weft knitted polyester) or a non-woven fabric could be used. The three-dimensional material can comprise a 3D spacer fabric material similar to the material described in International Publication WO 2013/175306 A2 and International Publication WO2014/020440.

In certain embodiments, the wound dressing 100 may incorporate or comprise one or more nitric oxide generating layers (e.g. a nitrite delivery layer, an acidic-group providing layer) as described herein this section or elsewhere in the specification. One of skill in the art will understand that the wound dressing 100 may incorporate any of the one or more nitric oxide generating layers disclosed herein this section or elsewhere in the specification. One of skill in the art will also understand that the one or more nitric oxide generating layers may be incorporated as a whole component layer or a part of a component layer. In some embodiments, the one or more nitric oxide generating layers may be provided below the transmission layer 226. In some embodiments, the one or more nitric oxide generating layers may be provided above the wound contact layer 222. In certain embodiments, the one or more nitric oxide generating layers may replace the transmission layer 226, such that the one or more nitric oxide generating layers are provided between an absorbent layer 221 (described further below) and the wound contact layer 222. In some embodiments, the one or more nitric oxide generating layers can supplement or replace the absorbent layer 221. In some embodiments, the wound dressing 100 does not have the wound contact layer 222, and the one or more nitric oxide generating layers may be the lowermost layer of the wound dressing 100. The one or more nitric oxide generating layers may have same or substantially similar size and shape with the transmission layer 226 and/or the absorbent layer 221. In some embodiments, the one or more nitric oxide generating layers or components thereof (e.g., a nitrite providing layer as described herein) can be separate of the wound dressing 100. For example, the one or more nitric oxide generating layers or components thereof can be provided as a separate layer that can be placed on a wound, and the wound dressing 100 can be placed thereupon.

The one or more nitric oxide generating layers may be constructed to be flexible but stiff enough to withstand negative pressure, such that the one or more nitric oxide generating layers is not collapsed excessively and thereby may transmit negative pressure sufficiently to the wound when negative pressure is supplied to the wound dressing 100. The one or more nitric oxide generating layers may be constructed to include sufficient number or size of pores to enable transmission of negative pressure. The one or more nitric oxide generating layer may include an aperture or hole, for example, under the port, to transmit negative pressure and/or wound fluid. Further, the one or more nitric oxide generating layers may have suitable thickness(es) to transmit suitable negative pressure to the wound. For example, the one or more nitric oxide generating layers may have a thickness of about 1 mm to 10 mm, or 1 mm to 7 mm, or 1.5 mm to 7 mm, or 1.5 mm to 4 mm, or 2 mm to 3 mm. In some embodiments, the one or more nitric oxide generating layers may have a thickness of approximately 2 mm.

In some embodiments, the layer 221 of absorbent material is provided above the transmission layer 226. The absorbent material, which can comprise a foam or non-woven natural or synthetic material, and which may optionally comprise a super-absorbent material, forms a reservoir for fluid, particularly liquid, removed from the wound site. In some embodiments, the layer 221 may also aid in drawing fluids towards the backing layer 220.

The material of the absorbent layer 221 may also prevent liquid collected in the wound dressing 100 from flowing freely within the dressing, and preferably acts so as to contain any liquid collected within the dressing. The absorbent layer 221 also helps distribute fluid throughout the layer via a wicking action so that fluid is drawn from the wound site and stored throughout the absorbent layer. This helps prevent agglomeration in areas of the absorbent layer. The capacity of the absorbent material must be sufficient to manage the exudates flow rate of a wound when negative pressure is applied. Since in use the absorbent layer experiences negative pressures the material of the absorbent layer is chosen to absorb liquid under such circumstances. A number of materials exist that are able to absorb liquid when under negative pressure, for example superabsorber material. The absorbent layer 221 may typically be manufactured from ALLEVYN^{™} foam, Freudenberg 114-224-4 or Chem-Posite^{™}11C-450. In some embodiments, the absorbent layer 221 may comprise a composite comprising superabsorbent powder, fibrous material such as cellulose, and bonding fibers. In a preferred embodiment, the composite is an air-laid, thermally-bonded composite.

In some embodiments, the absorbent layer 221 is a layer of non-woven cellulose fibers having super-absorbent material in the form of dry particles dispersed throughout. Use of the cellulose fibers introduces fast wicking elements which help quickly and evenly distribute liquid taken up by the dressing. The juxtaposition of multiple strand-like fibers leads to strong capillary action in the fibrous pad which helps distribute liquid. In this way, the super-absorbent material is efficiently supplied with liquid. The wicking action also assists in bringing liquid into contact with the upper cover layer to aid increase transpiration rates of the dressing.

An aperture, hole, or orifice 227 is preferably provided in the backing layer 220 to allow a negative pressure to be applied to the dressing 100. The fluidic connector 110 is preferably attached or sealed to the top of the backing layer 220 over the orifice 227 made into the dressing 100, and communicates negative pressure through the orifice 227. A length of tubing may be coupled at a first end to the fluidic connector 110 and at a second end to a pump unit (not shown) to allow fluids to be pumped out of the dressing. Where the fluidic connector is adhered to the top layer of the wound dressing, a length of tubing may be coupled at a first end of the fluidic connector such that the tubing, or conduit, extends away from the fluidic connector parallel or substantially to the top surface of the dressing. The fluidic connector 110 may be adhered and sealed to the backing layer 220 using an adhesive such as an acrylic, cyanoacrylate, epoxy, UV curable or hot melt adhesive. The fluidic connector 110 may be formed from a soft polymer, for example a polyethylene, a polyvinyl chloride, a silicone or polyurethane having a hardness of 30 to 90 on the Shore A scale. In some embodiments, the fluidic connector 110 may be made from a soft or conformable material.

Optionally, the absorbent layer 221 includes at least one through hole 228 located so as to underlie the fluidic connector 110. The through hole 228 may in some embodiments be the same size as the opening 227 in the backing layer, or may be bigger or smaller. As illustrated in Figure 2C a single through hole can be used to produce an opening underlying the fluidic connector 110. It will be appreciated that multiple openings could alternatively be utilized. Additionally, should more than one port be utilized according to certain embodiments of the present disclosure one or multiple openings may be made in the absorbent layer in registration with each respective fluidic connector. Although not essential to certain embodiments of the present disclosure the use of through holes in the super-absorbent layer may provide a fluid flow pathway which remains unblocked in particular when the absorbent layer is near saturation.

The aperture or through-hole 228 is preferably provided in the absorbent layer 221 beneath the orifice 227 such that the orifice is connected directly to the transmission layer 226 as illustrated in Figure 2C. This allows the negative pressure applied to the fluidic connector 110 to be communicated to the transmission layer 226 without passing through the absorbent layer 221. This ensures that the negative pressure applied to the wound site is not inhibited by the absorbent layer as it absorbs wound exudates. In other embodiments, no aperture may be provided in the absorbent layer 221, or alternatively a plurality of apertures underlying the orifice 227 may be provided. In further alternative embodiments, additional layers such as another transmission layer or an obscuring layer such as described with in International Patent Publication WO2014/020440, the entirety of which is hereby incorporated by reference, may be provided over the absorbent layer 221 and beneath the backing layer 220.

The backing layer 220 is preferably gas impermeable, but moisture vapor permeable, and can extend across the width of the wound dressing 100. The backing layer 220, which may for example be a polyurethane film (for example, Elastollan SP9109) having a pressure sensitive adhesive on one side, is impermeable to gas and this layer thus operates to cover the wound and to seal a wound cavity over which the wound dressing is placed. In this way, an effective chamber is made between the backing layer 220 and a wound site where a negative pressure can be established. The backing layer 220 is preferably sealed to the wound contact layer 222 in a border region around the circumference of the dressing, ensuring that no air is drawn in through the border area, for example via adhesive or welding techniques. The backing layer 220 protects the wound from external bacterial contamination (bacterial barrier) and allows liquid from wound exudates to be transferred through the layer and evaporated from the film outer surface. The backing layer 220 preferably comprises two layers; a polyurethane film and an adhesive pattern spread onto the film. The polyurethane film is preferably moisture vapor permeable and may be manufactured from a material that has an increased water transmission rate when wet. In some embodiments, the moisture vapor permeability of the backing layer increases when the backing layer becomes wet. The moisture vapor permeability of the wet backing layer may be up to about ten times more than the moisture vapor permeability of the dry backing layer.

The absorbent layer 221 may be of a greater area than the transmission layer 226, such that the absorbent layer overlaps the edges of the transmission layer 226, thereby ensuring that the transmission layer does not contact the backing layer 220. This provides an outer channel of the absorbent layer 221 that is in direct contact with the wound contact layer 222, which aids more rapid absorption of exudates to the absorbent layer. Furthermore, this outer channel ensures that no liquid is able to pool around the circumference of the wound cavity, which may otherwise seep through the seal around the perimeter of the dressing leading to the formation of leaks. As illustrated in Figure 2C, the absorbent layer 221 may define a smaller perimeter than that of the backing layer 220, such that a boundary or border region is defined between the edge of the absorbent layer 221 and the edge of the backing layer 220.

As shown in Figure 2C, one embodiment of the wound dressing 100 comprises an aperture 228 in the absorbent layer 221 situated underneath the fluidic connector 110. In use, for example when negative pressure is applied to the dressing 100, a wound facing portion of the fluidic connector may thus come into contact with the transmission layer 226, which can thus aid in transmitting negative pressure to the wound site even when the absorbent layer 221 is filled with wound fluids. Some embodiments may have the backing layer 220 be at least partly adhered to the transmission layer 226. In some embodiments, the aperture 228 is at least 1-2 mm larger than the diameter of the wound facing portion of the fluidic connector 11, or the orifice 227.

In particular for embodiments with a single fluidic connector 110 and through hole, it may be preferable for the fluidic connector 110 and through hole to be located in an off-center position as illustrated in Figure 2B. Such a location may permit the dressing 100 to be positioned onto a patient such that the fluidic connector 110 is raised in relation to the remainder of the dressing 100. So positioned, the fluidic connector 110 and the filter 214 may be less likely to come into contact with wound fluids that could prematurely occlude the filter 214 so as to impair the transmission of negative pressure to the wound site.

Similar to the embodiments of wound dressings described above, some wound dressings comprise a perforated wound contact layer with silicone adhesive on the skin-contact face and acrylic adhesive on the reverse. In some embodiments, the wound contact layer may be constructed from polyurethane, polyethylene or polyester. Above this bordered layer sits a transmission layer. Above the transmission layer, sits an absorbent layer. The absorbent layer can include a superabsorbent non-woven (NW) pad. The absorbent layer can over-border the transmission layer by approximately 5mm at the perimeter. The absorbent layer can have an aperture or through-hole toward one end. The aperture can be about 10 mm in diameter. Over the transmission layer and absorbent layer lies a backing layer. The backing layer can be a high moisture vapor transmission rate (MVTR) film, pattern coated with acrylic adhesive. The high MVTR film and wound contact layer encapsulate the transmission layer and absorbent layer, creating a perimeter border of approximately 20 mm. The backing layer can have a 10 mm aperture that overlies the aperture in the absorbent layer. Above the hole can be bonded a fluidic connector that comprises a liquid-impermeable, gas-permeable semi-permeable membrane (SPM) or filter that overlies the aforementioned apertures.

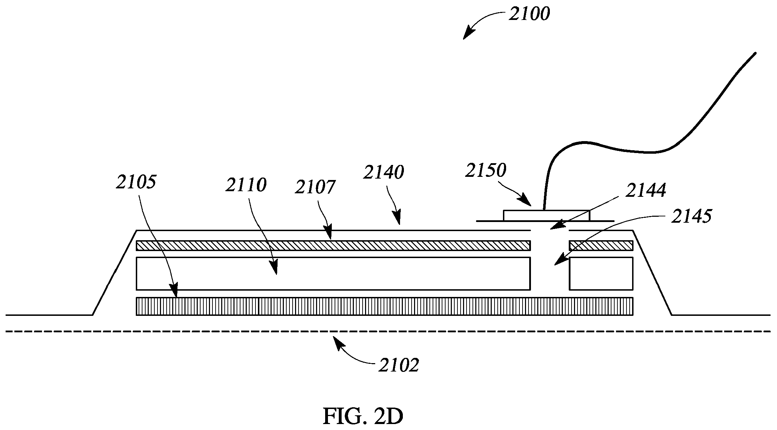

Figure 2D depicts an embodiment of a wound dressing, similar to the wound dressings of Figures 2A-2C. With reference to Figure 2D, a masking or obscuring layer 2107 can be positioned beneath at least a portion of the backing layer 2140. In some embodiments, the obscuring layer 2107 can have any of the same features, materials, or other details of any of the other embodiments of the obscuring layers disclosed herein, including but not limited to having any viewing windows or holes. Examples of wound dressings with obscuring layers and viewing windows are described in International Patent Publication WO2014/020440. Additionally, the obscuring layer 2107 can be positioned adjacent to the backing layer, or can be positioned adjacent to any other dressing layer desired. In some embodiments, the obscuring layer 2107 can be adhered to or integrally formed with the backing layer. Preferably, the obscuring layer 2107 is configured to have approximately the same size and shape as the absorbent layer 2110 so as to overlay it. As such, in these embodiments the obscuring layer 2107 will be of a smaller area than the backing layer 2140.

Preferably the absorbent layer 2110 and the obscuring layer 2107 include at least one through hole 2145 located so as to underlie the port 2150. Of course, the respective holes through these various layers 2107, 2140, and 2110 may be of different sizes with respect to each other. As illustrated in Figure 2D a single through hole can be used to produce an opening underlying the port 2150. In certain embodiments, the port may be replaced with or used in combination with a fluidic connector such as depicted in Figure 2C. It will be appreciated that multiple openings could alternatively be utilized. Additionally, should more than one port be utilized according to certain embodiments of the present disclosure one or multiple openings may be made in the absorbent layer and the obscuring layer in registration with each respective port. Although not essential to certain embodiments of the present disclosure the use of through holes in the super-absorbent layer may provide a fluid flow pathway which remains unblocked in particular when the absorbent layer 2110 is near saturation.

The aperture or through-hole 2144 may be provided in the absorbent layer 2110 and the obscuring layer 2107 beneath the orifice 2144 such that the orifice is connected directly to the transmission layer 2105. This allows the negative pressure applied to the port 2150 to be communicated to the transmission layer 2105 without passing through the absorbent layer 2110. This ensures that the negative pressure applied to the wound site is not inhibited by the absorbent layer as it absorbs wound exudates. In other embodiments, no aperture may be provided in the absorbent layer 2110 and/or the obscuring layer 2107, or alternatively a plurality of apertures underlying the orifice 2144 may be provided.

In some embodiments, the obscuring layer 2107 can help to reduce the unsightly appearance of a dressing during use, by using materials that impart partial obscuring or masking of the dressing surface. The obscuring layer 2107 in one embodiment only partially obscures the dressing, to allow clinicians to access the information they require by observing the spread of exudate across the dressing surface. The partial masking nature of this embodiment of the obscuring layer enables a skilled clinician to perceive a different color caused by exudate, blood, by-products etc. in the dressing allowing for a visual assessment and monitoring of the extent of spread across the dressing. However, since the change in color of the dressing from its clean state to a state containing exudate is only a slight change, the patient is unlikely to notice any aesthetic difference. Reducing or eliminating a visual indicator of wound exudate from a patient's wound is likely to have a positive effect on their health, reducing stress for example.

In some embodiments, the obscuring layer can be formed from a non-woven fabric (for example, polypropylene), and may be thermally bonded using a diamond pattern with 19% bond area. In various embodiments, the obscuring layer can be hydrophobic or hydrophilic. Depending on the application, in some embodiments, a hydrophilic obscuring layer may provide added moisture vapor permeability. In some embodiments, however, hydrophobic obscuring layers may still provide sufficient moisture vapor permeability (i.e., through appropriate material selection, thickness of the obscuring layer), while also permitting better retention of dye or color in the obscuring layer. As such, dye or color may be trapped beneath the obscuring layer. In some embodiments, this may permit the obscuring layer to be colored in lighter colors or in white. In the preferred embodiment, the obscuring layer is hydrophobic. In some embodiments, the obscuring layer material can be sterilizable using ethylene oxide. Other embodiments may be sterilized using gamma irradiation, an electron beam, steam or other alternative sterilization methods. Additionally, in various embodiments the obscuring layer can colored or pigmented, e.g., in medical blue. The obscuring layer may also be constructed from multiple layers, including a colored layer laminated or fused to a stronger uncolored layer. Preferably, the obscuring layer is odorless and exhibits minimal shedding of fibers.

### Multi-Layered Dressing for Use Without Negative Pressure

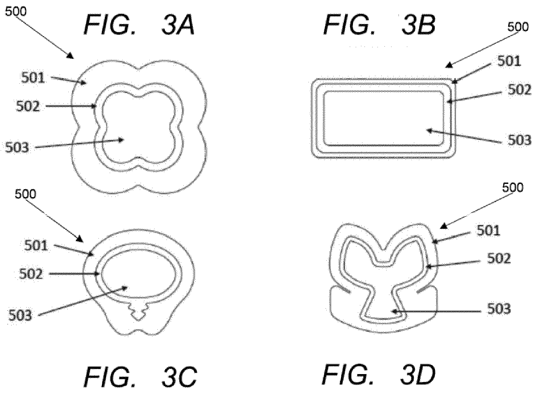

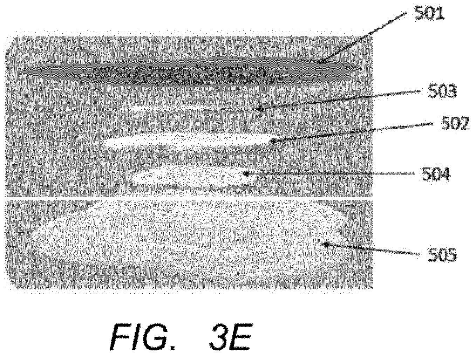

Figures 3A-3D illustrates various embodiments of a wound dressing 500 that can be used for healing a wound without negative pressure. Figure 3E illustrates a cross-section of the wound dressing in Figures 3A-3D. As shown in the dressings of Figures 3A-3E, the wound dressings can have multiple layers similar to the dressings described with reference to Figures 2A-2D except the dressings of Figures 3A-3E do not include a port or fluidic connector. The wound dressings of Figures 3A-3E include a cover layer 501 and an optional wound contact layer 505 as described herein. In some embodiments, the cover layer 501 may be permeable to moisture and/or air. The wound dressing can include various layers positioned between the wound contact layer 505 and cover layer 501. For example, the dressing can include one or more absorbent layers or one or more transmission layers as described herein with reference to Figures 2A-2D.

As shown in Figures 3A-3E, the dressing 500 may include a perforated wound contact layer 505 and a top film 501. Further components of the wound dressing 500 include a foam layer 504, such as a layer of polyurethane hydrocellular foam, of a suitable size to cover the recommended dimension of wounds corresponding to the particular dressing size chosen. An optional layer of activated charcoal cloth (not shown) of similar or slightly smaller dimensions than layer 504 may be provided to allow for odour control. An absorbent layer 502, such as a layer of superabsorbent air-laid material containing cellulose fibres and a superabsorbent polyacrylate particulates, is provided over layer 504, of dimensions slightly larger than layer 504, and allows for an overlap of superabsorbent material and acts as leak prevention. A masking or obscuring layer 503, such as a layer of three-dimensional knitted spacer fabric, is provided over layer 502, providing protection from pressure, while allowing partial masking of the top surface of the superabsorber where coloured exudate would remain. In this embodiment this is of smaller dimension (in plan view) than the layer 502, to allow for visibility of the edge of the absorbent layer, which can be used by clinicians to assess whether the dressing needs to be changed.

The wound dressing 500 may incorporate or comprise one or more nitric oxide generating layers (e.g. a nitrite delivery layer, an acidic-group providing layer) as described herein this section or elsewhere. One of skill in the art will understand that the wound dressing 500 may incorporate any of the one or more nitric oxide generating layers disclosed herein this section or elsewhere in the specification. One of skill in the art will also understand that the one or more nitric oxide generating layers may be incorporated as a whole component layer or a part of a component layer. In some embodiments, the nitric oxide generating layers may be provided below the cover layer 501. In some embodiments, the one or more nitric oxide generating layers may be provided above the wound contact layer 505. In certain embodiments, the dressing 500 may not include the wound contact layer 505, such that one of the nitric oxide generating layers may be the lowermost layer and be configured to touch the wound surface. In some embodiments, the one or more nitric oxide generating layers may be provided below the foam layer 504. In embodiments, the one or more nitric oxide generating layers may replace the foam layer 504. In some embodiments, the dressing 500 may include only the cover layer 501 and the one or more nitric oxide generating layers. In some embodiments, the one or more nitric oxide generating layers or components thereof (e.g., a nitrite providing layer as described herein) can be separate of the wound dressing 500. For example, the one or more nitric oxide generating layers or components thereof can be provided as a separate layer that can be placed on a wound, and the wound dressing 500 can be placed thereupon.

As described previously herein, the one or more nitric oxide generating layers, may be incorporated into or used with commercially available dressings, such as ALLEVYN^{™} foam, ALLEVYN^{™} Life, ALLEVYN^{™} Adhesive, ALLEVYN^{™} Gentle Border, ALLEVYN^{™} Gentle, ALLEVYN^{™} Ag Gentle Border, ALLEVYN^{™} Ag Gentle, Opsite Post-Op Visible. In some embodiments, the wound dressing 500 may include the cover layer 501, the wound contact layer 505 and the nitric oxide generating layers sandwiched therebetween. In some embodiments, the wound dressing 500 may include the cover layer 501, the absorbent layer 502, the nitric oxide generating layers below the absorbent layer 502, and the wound contact layer 505.

Further details regarding wound dressings that may be combined with or be used in addition to the embodiments described herein, are found in U.S. Patent No. 9,877,872, issued on January 30, 2018, titled "WOUND DRESSING AND METHOD OF TREATMENT," including further details relating to embodiments of wound dressings, the wound dressing components and principles, and the materials used for the wound dressings.

### Multilayered Wound Dressing with an Integrated Source of Negative Pressure

In some embodiments, a source of negative pressure (such as a pump) and some or all other components of the TNP system, such as power source(s), sensor(s), connector(s), user interface component(s) (such as button(s), switch(es), speaker(s), screen(s), etc.) and the like, can be integral with the wound dressing, such as the dressings described above in relation to Figures 1-3D. Additionally, some embodiments related to wound treatment comprising a wound dressing described herein may also be used in combination or in addition to those described in International Application WO 2016/174048 and International Patent Application PCT/EP2017/055225, filed on March 6, 2017, entitled "WOUND TREATMENT APPARATUSES AND METHODS WITH NEGATIVE PRESSURE SOURCE INTEGRATED INTO THE WOUND DRESSING," including further details relating to embodiments of wound dressings, the wound dressing components and principles, and the materials used for the wound dressings and wound dressing components.

In some embodiments, the pump and/or other electronic components can be configured to be positioned adjacent to or next to the absorbent and/or transmission layers in the wound dressing so that the pump and/or other electronic components are still part of a single apparatus to be applied to a patient with the pump and/or other electronics positioned away from the wound site.

### Nitric Oxide Generating Layers

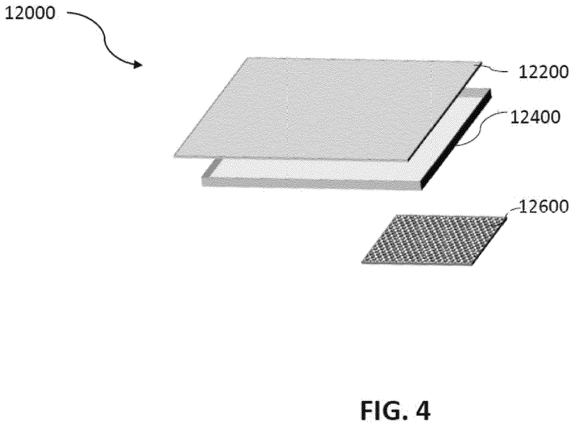

FIGS. 4-5 illustrate a wound dressing 12000 including nitric oxide generating layers according to some embodiments. In the illustrated embodiments, the wound dressing 12000 includes a cover layer 12200, an activator layer 12400, and a nitric oxide source layer 12600. In some embodiments, the wound dressing 12000 may include additional layers, as further described herein. One of skill in the art will understand that although the various sections of the dressing may be referred to as "layers," such sections may be in other suitable shapes or configurations. As will be understood by one of skill in the art, the wound dressing and/or nitric oxide delivering embodiments described herein this section or elsewhere in the specification may be applied over a wound and/or over the surrounding skin, such as the periwound area.

The cover layer 12200 may be gas impermeable, but moisture vapor permeable, and can extend across the width of the wound dressing 12000. The cover layer 12200, which may for example be a polyurethane film (for example, Elastollan SP9109 or Elastollan SP806) having a pressure sensitive adhesive on one side, may be impermeable to gas and this layer may thus operate to cover the wound and to seal a wound cavity over which the wound dressing is placed. Therefore, a chamber or a sealed wound space is made between the cover layer 12200 and the wound site. In some embodiments, negative pressure can be established within the chamber or the sealed wound space made between the cover layer 12200 and the wound site. The cover layer 12200 protects the wound from external bacterial contamination (bacterial barrier) and allows liquid from wound exudates to be transferred through the layer and evaporated from the film outer surface. The cover layer 12200 may include two or more layers, for example, a polyurethane film and an adhesive pattern spread onto the film. In certain examples, the polyurethane film may be moisture vapor permeable and may be manufactured from a material that has an increased water transmission rate when wet. In some embodiments, the moisture vapor permeability of the cover layer increases when the cover layer becomes wet. The moisture vapor permeability of the wet cover layer may be up to about ten times more than the moisture vapor permeability of the dry cover layer. In some embodiments, the cover layer 12200 may be replaced or supplemented with an additional wound dressings described elsewhere herein, such that the additional wound dressings are positioned above the nitric oxide generating layers. The cover layer may also be shower proof, such that a dressing incorporating such a cover layer may be used in the shower. The cover layer may be configured such that nitric oxide does not immediately escape through the cover layer, meaning that the cover layer is nitric oxide impermeable or semi-impermeable, thereby trapping nitric oxide against the tissue such that nitric oxide can interact with the body of a user. One of skill in the art will understand that the cover layer may be made to be both vapor permeable, but nitric oxide impermeable.

The nitric oxide source layer 12600 may provide one or more nitric oxide-releasing agents at the wound site. The nitric oxide-releasing agent can include any chemical entity that yields nitric oxide at the wound site when activated or otherwise stimulated to do so. In some embodiments, the nitric oxide-releasing agent can include nitrite ion, a nitrite salt, organic and inorganic nitrites, or any pharmacologically acceptable source of nitrite such that the nitrite ion can be reduced to produce nitric oxide at the wound site. For example, the nitric oxide source layer 12600 and/or element may include one or more of ammonium nitrite, lithium nitrite, calcium nitrite, sodium nitrite, potassium nitrite. In some embodiments, the nitric oxide source layer may be a suitable material layer or element that includes alkali metal nitrites and/or alkaline earth metal nitrites. In certain embodiments, the nitrites may include: LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, Ra(NO₂)₂ or any other suitable nitrite. In some embodiments, a precursor of nitrite ions, such as nitrous acid, nitrate ions, nitroprusside ions, or any pharmacologically acceptable salts thereof may be used as the source of the nitrite. In some embodiments, the nitric oxide-releasing agents may include nitrites such as nitro-functionalized compounds. For example, the nitric oxide-releasing agents may include nitroglycerine, isoamyl nitrite, isorbide mononitrate, N-(Ethoxycarbonyl)-3-(4-morpholinyl)sydnoneimine; 3-morpholinosydnonimine; 1,2,3,4-Oxatriazolium; 5-amino-3-(3,4-di-chlorophenyl)-chloride; 1,2,3,4-Oxatriazolium; 5-amino-3-(chloro-2-methyl-phenyl)chloride; 1,2,3,4-Oxatriazolium, 3-(3-chloro-2-methylphenyl)-5-[[[cyanomethylamino]carbonyl]amino]-hydroxide inner salt; S-nitroso-N-acetyl-(D,L)-penicillamine; l-[(4',5'-Bis(carboxymethoxy)-2l-nitrophenyl)methoxy]-2-oxo-3,3,diethyl-1-triazene dipotassium salt; and [1-(4', 5'-Bis(carboymethoxy)-2'-nitropheyl)methoxy]-2-oxo-3,3-diethyl-1-triazine diacetoxymethyl ester.

In some embodiments, the nitric oxide-releasing agent of the nitric oxide source layer 12600 can include diazeniumdiolates, including O-alkylated diazeniumdiolate, O-derivatized diazeniumdiolate, and non-O-derivatized diaziniumdiolate. For example, the nitric oxide-releasing agent can include diethylamine/NO, V-PYRRO/NO and/or Spermine/NO. In some embodiments, the nitric oxide-releasing agent of the nitric oxide source layer 12600 can include S-nitrosothiols, such as S-nitro-gluthathione, S-nitroso-N-acetylcystein, S-nitroso-acetylpenicillamine. In some embodiments, the nitric oxide-releasing agent of the nitric oxide source layer 12600 may include silica, or silica nano-particles modified with nitric oxide. In some embodiments, the nitric oxide-releasing agent can be a polymer modified with nitric oxide to include nitric oxide. For example, polyethyleneimine, polypropyleneimines, polybutyleneimines, polyurethanes or polyamides can be modified with nitric oxide to form diazeniumdiolate. In some embodiments, the nitric oxide source layer 12600 may be constructed from such polymers modified with nitric oxide. Further examples of the nitric oxide-releasing agents are provided in International Publication No. WO 2006/058318, and Liang et al., "Nitric oxide generating/releasing materials", Future Science OA, 1 (1) (2015),

In some embodiments, the nitric oxide source layer 12600 may include a nitric oxide-releasing agent (e.g. sodium nitrite) in an aqueous solution. For example, the nitric oxide source layer 12600 may include a material imbibed with the nitric oxide-releasing agent (e.g. sodium nitrite) solution. In some embodiments, the nitric oxide source layer 12600 may include a dry nitric oxide-releasing agent (e.g. sodium nitrite) in solid form.

The nitric oxide source layer 12600 may include a mesh, a foam, a gel or any other material suitable for containing the nitric oxide-releasing agent. For example, the nitric oxide source layer 12600 may include a mesh imbibed with the nitric oxide-releasing agent (e.g. sodium nitrite) solution. The mesh may be knitted, woven or non-woven. The mesh may be made of a polymeric material, for example, viscose, polyamide, polyester, polypropylene or a combination thereof. In some embodiments, the nitric oxide source layer 12600 may include polypropylene, polyester, polyurethane, polyvinyl chloride, polyamide, viscose, polyester, polypropylene and/or cellulose. As described herein, the nitric oxide source layer 12600 may be constructed from one or more polymers modified with nitric oxide. The nitric oxide source layer 12600 could also be made of a hydrogel without acidic groups to prevent reaction with nitrite ions to emit nitric oxide. In some embodiments, the nitric oxide source layer 12600 may be constructed from a colored material, such that the nitric oxide source layer 12600 can be visible to assist positioning of the wound dressing 12000 during application to the wound, and to reduce the risk of incomplete removal of the nitric oxide source layer 12600 from the wound after treatment. The nitric oxide source layer 12600 may be fully or semi-permeable to the diffusion of nitric oxide.

In some embodiments, the nitric oxide source layer 12600 is the lowermost layer of the dressing 12000, such that the nitric oxide source layer 12600 may contact the wound. In some embodiments, the nitric oxide source layer 12600 may be positioned within and/or over the wound. The nitric oxide source layer may be constructed such that the nitric oxide source layer 12600 do not substantially adhere to the skin or wound, or cause da mage to the wound when in contact with the wound. In some embodiments, the dressing 12000 may include one or more layers, for example a wound contact layer, beneath the nitric oxide source layer 12600. In some embodiments, the wound dressing 12000 may include two or more nitric oxide source layers. For example, the wound dressing 12000 may include 2, 3, 4, 5, 6, 7 or more nitric oxide source layers. In some embodiments, the nitrite providing layer 12600 can be separate of the wound dressing 12000. For example, the nitrite providing layer 12600 can be provided as a separate layer that can be placed on a wound, and the wound dressing 12000 can be placed thereupon. The nitric oxide-releasing agent may be incorporated into the nitrite providing layer to provide a nitrite dose (e.g., a sodium nitrite dose), in M (Molar) of about: 0.01 to 5.0, 0.5 to 4.5, 1.0 to 3.0, 1.0 to 2.0, and/or 1.0 to 1.5. For example, the dose may be about 0.50 M, about 0.01 M, about 1.5 M, about 2 M, or about 2.5 M.

The activator layer 12400 may contain chemical agents, functional groups or moieties which can activate and/or facilitate release of nitric oxide from the nitric oxide-releasing agent. For example, protons or acidic environment promotes the reduction of nitrites to nitric oxide, and the activator layer 12400 may include acidic groups or moieties which may provide protons in aqueous environment, thereby lowering the pH at the site of application. In certain embodiments, the acidic groups or moieties are immobilized at the activator layer 12400, for example on the surface of the activator layer 12400. The acidic groups or moieties may be covalently bonded at the activator layer 12400. In some embodiments, the activator layer 12400 may include an acidic solution. The activator layer 12400 may include a mesh, a foam, a gel or any other material suitable for containing acid groups or moieties. In embodiments, the activator layer 12400 is positioned above the nitric oxide source layer 12600 or the activator layer 12400 may be positioned below the nitric oxide source layer 12600. In some embodiments, the activator layer 12400 may include proton sources such as water, methanol, ethanol, propanols, butanols, pentanols, hexanols, phenols, naphtols or polyols; aqueous acidic buffers such as phosphates, succinates, carbonates, acetates, formats, propionates, butyrates, fatty acids, amino acids, or ascorbic acids; or any suitable enzymatic or catalytic compounds. In some embodiments, body fluid such as blood, lymph, bile, or wound exudate may function as the activator, and can assist the activator layer 12400. Also disclosed are wound dressings 12000 which may not include the activator layer 12400, and wound fluid or wound exudate may function as the activator. Further examples of the activators for the nitric oxide-releasing agents are provided in International Publication No. WO 2006/058318, and Liang et al., "Nitric oxide generating/releasing materials", Future Science OA, 1 (1) (2015).

In some embodiments, the wound dressing 12000 may include two or more nitric oxide source layers and/or two or more activator layers. For example, the wound dressing 12000 may include 2, 3, 4, 5, 6, 7 or more nitric oxide source layers and/or activator layers.

In some embodiments, the activator layer 12400 includes hydrogel, such that the activator layer 12400 can absorb the wound exudate. In certain examples, the activator layer 12400 may be constructed of a xerogel. The activator layer 12400 may be constructed from any suitable materials disclosed herein. The gel of the activator layer 12400 may be presented in different physical formats. For example, the activator layer 12400 may be foamed during curing. The hydrogel may be poured into a foam and then cured in the foam. In some embodiments, the activator layer 12400 may be perforated through its thickness. The perforations may be sized to allow fluid absorption and for the desired therapeutic dose of nitric oxide to be released from the wound dressing. For example, the perforations may have a diameter sized approximately between 0.1 mm and 10 mm, between 0.15 mm and 7 mm, between 0.2 mm and 5 mm, between 0.5 mm and 4 mm or between 0.7 mm and 3 mm. The perforations may have a circular shape, a square shape, a triangular shape, or any other suitable shape. The foamed construction and/or the perforations may contribute to fluid handling capabilities of the activator layer.

In some embodiments, an activator material for the activator layer may be provided as a dispensable composition, for example as a prepolymer solution or otherwise malleable form, instead of being provided as the activator layer such as the activator layer 12400, such that it can be applied over the wound and/or around the wound more freely. For example, the activator material may be provided as gel prepolymer solution, such that it can be applied closely to or around a wound having an irregular shape size by a clinician. In some embodiments, the activator material, such as the gel prepolymer solution, may be provided in and/or applied with a syringe, and the gel prepolymer solution may have a viscosity suitable to be dispensed from the syringe. The activator material can be also formulated such that it can be rapidly cured and no longer flows once applied to or around the wound. The activator material may include an evaporative solvent, such as isopropanol. The activator material can have a suitable secondary curing mechanism, such as photoinitiated acrylate functionality. In some embodiments, the activator material can be provided as a reactive two-part system. For example, a first part and a second part may be provided to be mixed to result in polymer formation immediately before dispensing. In some embodiments, the first part and the second part may be oppositely charged flowable gels, such that they can interact on mixing to provide gels that do not flow substantially. In some embodiments, the activator material may include a material such as a gel which change in response to the change in environment. For example, the activator material may include a material such as certain pluronics, such that it can be cured once the temperature changes as being applied from the dispenser or syringe to the skin. The activator material may be applied such that it can interact with nitrite from the nitric oxide source layer 12600 (which may provide nitrite) to generate nitric oxide. Once the activator material is applied and cured or does not flow otherwise, the cover layer 18200 may be applied.

Once the dressing 12000 is activated, for example by placing the activator layer 12400 in contact with the nitric oxide source layer 12600, nitric oxide-releasing agents from the nitric oxide source layers 12600 releases nitric oxide. For example, in some embodiments, nitrites can be reduced to nitric oxide in the presence of an acidic environment provided by the activator layer 12400 as shown below:

NO₂⁻ + H⁺ ⇄ HNO₂ (1)

2HNO₂ ⇄ H₂O + N₂O₃ (2)

N₂O₃ ⇄ NO + NO₂ (3)

The activator layer 12400 and the nitric oxide source layer 12600 may be positioned such that the nitric oxide-releasing agents can react to provide nitric oxide. For example, the activator layer 12400 and the nitric oxide source layer 12600 may be in contact with each other within the dressing 12000 when in use. In some embodiments, one or more additional layers may be positioned between the activator layer 12400 and the nitric oxide source layer 12600. In some embodiments, the activator layer 12400 and the nitric oxide source layer 12600 may be fluidically isolated from each other before applying the dressing 12000 to the patient to prevent premature release of nitric oxide. For example, the nitric oxide source layer 12600 may be provided in a packaging separate from the rest of the dressing 12000. Once the dressing 12000 is activated, the nitric oxide-releasing agents from the nitric oxide source layer 12600 may disperse within the dressing 12000. In some embodiments, the nitric oxide-releasing agents may be dissolved in wound exudate and wound exudate may facilitate dispersal of the nitric oxide-releasing agents. At least a portion of the nitric oxide-releasing agents would react to release nitric oxide in the presence of the activators of the activator layer 12400. The generated nitric oxide may diffuse into the wound or be delivered to the wound by any suitable mechanisms. In some embodiments, the generated nitric oxide may not be delivered immediately or at all and is instead held within the dressing, for example by a selectively permeable membrane, such that the nitric oxide may prevent growth of or kill microbes within the dressing.

In some embodiments, the wound dressing 12000 can include a reducing agent to facilitate reduction of the nitric oxide-releasing agent (e.g. nitrite ion) to nitric oxide. Physiologically acceptable examples of such reducing agents include but are not limited to: iodide anion, ascorbic acid, ascorbate (e.g. sodium ascorbate), isoascorbates (e.g. sodium isoascorbate), hydroquinone, butylated quinone, tocopherol, butylated hydroquinone, hydroquinone varients, butylated hydroxyanisole, butylated hydroxytoluene, beta-carotene, potassium iodide, ascorbate variants, iso-ascorbate variants, any other suitable reducing agent, and/or any of the antioxidants and/or reducing agents described herein. The reducing agent may be included in one or more layers of the wound dressing 12000. For example, the reducing agent may be included in the cover layer 12200, the activator layer 12400, the nitric oxide source layer 12600, the wound contact layer 12800, and/or any suitable layers of nitric oxide generating wound dressings described herein. The reducing agent may be incorporated to the one or more layers, for example, by physical entrapment, physical blending, coating, covalent bonding, or any other suitable methods. The reducing agent may be incorporated into the dressing and/or into the appropriate layer, such as a hydrogel activating layer, at a w/w % of about: 0.01 to 5.0%, 0.1 to 4.5%, 1.0 to 3.0%, 1.0 to 1.5%, and/or 1.5 to 2.5%. For example, the w/w% may be about 0.02%, about 0.03%, about 0.8%, about 1.2%, about 1.4%, or about 2.43%. Higher levels of reducing agent may lead to increased production of nitric oxide; however, very high levels of reducing agent may become toxic.

As described herein, the nitric oxide source layer may include nitrite and may be referred to as a nitrite delivery layer or a nitrite providing layer in this specification. As described herein, the activator layer may include acids and may be referred to as an acid providing layer or an acid delivery layer in this specification. The nitric oxide source layer/the nitrite delivery layer/the nitrite providing layer and the activator layer/the acid providing layer may be collectively or individually referred to as nitric oxide generating layer(s) in this specification. As will be understood by one of skill in the art, the nitrite providing layers disclosed herein may be prepared and provided separate from the wound dressing. For example, the nitrite providing layer may be applied to the wound and the wound dressing may be applied above the nitrite providing layer.

### Nitric Oxide Dressing Materials and Construction

As will be understood by one of skill in the art, the materials and dressing constructions described above in relation to the nitric oxide delivery dressings 1200 of Figures 4- 5 and elsewhere in the specification may include multiple suitable constructions and different types of materials. For example, the topmost layer furthest away from the wound may be a top or cover film layer, such as a top or cover layer disclosed herein, such as polyurethane materials. Such a top or cover film may be construction from materials used in the cover layer of the RENASYS drape, sold by Smith + Nephew. For example, in some embodiments the cover layer can be an IV3000 top film. Below the top or cover film layer may be a masking or fabric layer, which may be constructed of any suitable material disclosed as a masking or fabric layer herein. The masking layer may be constructed from a stretch and non-stretch polyester, polyethylene, polypropylene, polypropylethylene, and nonwovens and suitable blends constructed thereof. For example, in some embodiments the masking layer can be a 17 gsm polypropylene mask layer. Further suitable nonwovens and blend may also be utilized. In certain embodiments, the masking layer may be foam. Beneath the masking or fabric layer is an activator layer, similar to the activator layers described herein and throughout the specification. Such an activator layer may be constructed from a hydrogel adhesive, optionally containing a central polyester supporting mesh and/or supporting release liners. For example, in some embodiments the activator layer can be a DURAFIBER format loaded with a hydrogel as described herein. In such an example, depending on the dressing size, an equivalent of about 6 grams of hydrogel can be loaded onto a 10.8 cm x 10.8 cm piece of DURAFIBER, which can be cut down to 10 cm x 10 cm resulting in about 5.14 grams of the hydrogel on the 10 cm x 10 cm piece of DURAFIBER. The activator layer may be constructed from any suitable hydrogel material disclosed herein such as an acrylic acid hydrogel and/or a sulfonic acid hydrogel. Below the activator layer may be an acquisition distribution layer, which may be constructed of any suitable acquisition distribution layer materials disclosed herein, such as in relation to Figures 2C-2D. For example, the acquisition distribution layer may be constructed from 3-D knit, gauze and/or stretch polyester fibers woven into a net format, similar to the material used in Acticoat Flex by Smith + Nephew, although silver is optional. In some embodiments the acquisition distribution layer may be constructed from a pre-polymer solution with a mixture of water, surfactant, and polyethylene glycol such as foams used in Allevyn foam by Smith + Nephew. The masking layer and acquisition distribution layer may use the same materials and be interchangeable. In certain embodiments, the acquisition distribution layer may be pressed into the activator layer and/or cured into the activator layer. Curing the acquisition distribution layer into the activator layer may increase the rate of nitric oxide formation due to more rapid transport. Under the acquisition distribution layer, there may be a wound contact layer which may be constructed from any suitable material disclosed herein, such as in relation to Figures 2C-2D. For example, the wound contact layer may include a silicone adhesive and perforated polyurethane film. The wound contact layer may include an acrylic adhesive. A nitric oxide source layer, such as a nitrite layer, constructed from any suitable materials disclosed herein, may be positioned beneath the wound contact layer such that the nitric oxide source layer is directly against a wound or other tissue. In some embodiments, the nitric oxide source layer may be in other positions, such as above the activator layer and/or elsewhere in the dressing. For example, in some embodiments the nitrite providing layer can be a separate 17 gsm polypropylene mesh saturated with a sodium nitrite solution. In certain, embodiments the ALLEVYN or PICO dressings disclosed in Figures 2-3 may be placed directly over an activator layer and underlying nitric oxide source layer. Placing the nitric oxide source layer directly against the wound, periwound area, and/or other tissues may allow for increased release of nitric oxide directly into the tissue. As will be understood by one of skill in the art, the wound dressing and/or nitric oxide delivering embodiments described herein this section or elsewhere in the specification may be applied over a wound and/or over the surrounding skin, such as the periwound area.

### Chemiluminescence

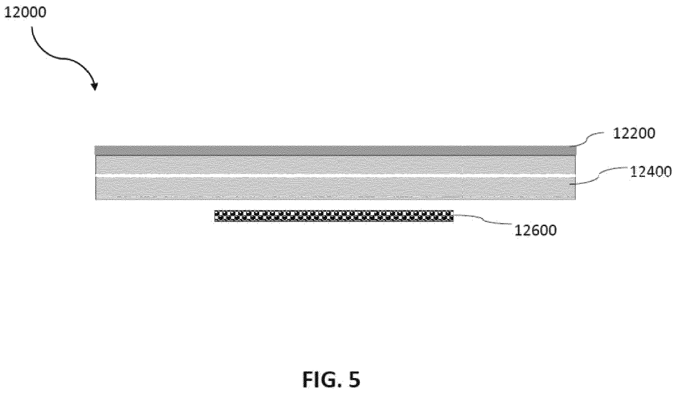

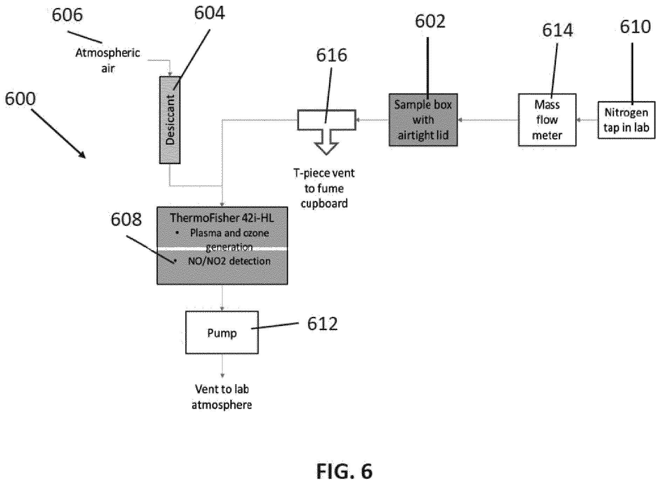

Figure 6 shows an example setup 600 for a chemiluminescence protocol for testing a nitric oxide delivery dressing such as disclosed above in relation to Figures 4 and 5. The protocol may include a sample 602, desiccant 604, an atmospheric air source 606, a chemiluminescence detector 608, a nitrogen supply 610, an air pump 612, a mass flow meter 614, and T-piece connector 616. In certain embodiments, a ThermoFisher 42i-HL detector may be used as a chemiluminescence detector 608. After warming up the equipment with air flow under atmospheric pressure, the sample box 602 and nitrogen supply can be connected to the equipment. The nitrogen flow through the mass flow controller may be set to a suitable value, such as between about: 1 to 100, 10 to 90, 25 to 75, 40 to 60, or about 50 mL/min. After flushing the system (such as for about 1 to 60, 10 to 50, 20 to 40, or about 30 minutes), a nitric oxide source layer (such as a nitrite mesh) and activator layer (such as an acid providing hydrogel) may be placed in the sample chamber 602. In embodiments, the nitrite mesh is smaller in total area than the activator layer. In particular embodiments, the nitric oxide source layer and/or the activator layer may have a length and/or a width of about .5 to 20, 1 to 10, 2 to 8, or about 4 to 6 centimeters. In certain embodiments, the nitric oxide source layer may be 2.5 cm x 2.5 cm while the activator layer is 3 cm x 3 cm.

NO/NO₂ release concentrations may be measured by the chemiluminescence detector at an appropriate rate, checking the concentrations in ppb or ppm and monitoring periodically, such as about every 1, 2, 5, 10, 30, 60 or 90 seconds. In certain embodiments, the NO/NO₂ concentration may be checked in ppm.

As will be understood by one of skill in the art, maximizing NO over NO₂ is desirable for the dressings disclosed herein, such as the dressings described in relation to Figures 4-5. While nitrogen dioxide (NO₂) may exert antimicrobial properties, NO₂ does not have the vasodilating properties nor the capability of activating cell proliferation of NO. It is therefore generally desirable to reduce the generation of NO₂ as far as possible in the acidification of nitrites such as by such means as reducing the oxidation of dissolved nitric oxide (NO) by removing the oxygen from the body of the hydrogel where the acidification of nitrite takes place. The nitric oxide delivery dressings disclosed herein may produce both NO and NO₂. In some embodiments, the nitric oxide dressings disclosed herein may produce NO and NO₂ in a ratio of NO/ NO₂ such as about 0.5:1 to 500:1, 1:1 to 400:1, 10:1 to 300:1, 20:1 to 200:1. 50:1 to 100:1. For example, the ratio may be about or at least about 0.5:1 1.01:1, 1.1:1, 1:1, 2:1. 5:1, 10:1, 20:1, 30:1, 50:1, 100:1, 200:1, or 500:1.

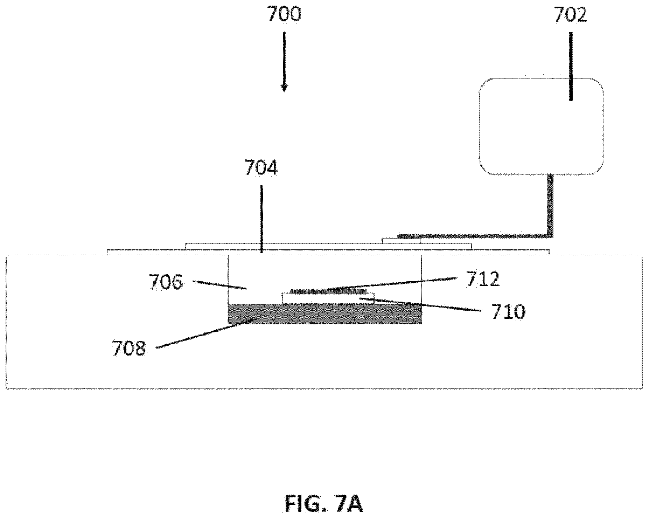

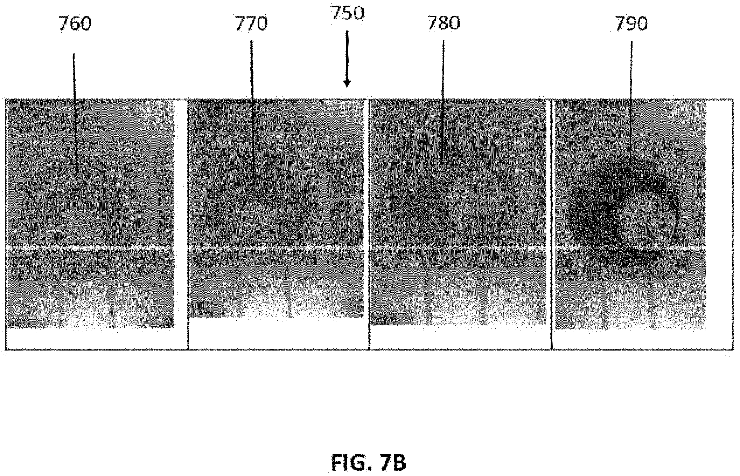

Figures 7A-B show an example of an experimental set-up 700 and the subsequent results 750 demonstrating nitric oxide delivery from a combination of activator layer and nitric oxide source layer, similar to the dressings described in relation to Figures 4 and 5, while under negative pressure. As shown in Figure 7A, a negative pressure wound therapy pump 702 is connected to a negative pressure wound therapy dressing 704 such as described herein in Figures 2A-2D. The dressing is sealed over a chamber 706 containing nitrite test solution 708 which changes color in the presence of NO. Figure 7B shows an example of results of the negative pressure nitric oxide experiment shown in Figure 7A. Prior to applying negative pressure, the test solution did not change color 750. After running negative pressure for a period of time to ensure that no background color change occurred as shown in 760, an activator layer 710 such as described herein (such as an acid-providing hydrogel), was placed in the chamber and negative pressure was applied. Again, no color change occurred 770. Lastly, a nitric oxide source layer 712 such as described herein (such as a sodium nitrite mesh) was placed onto the activator layer 780 without having the nitric oxide source layer touch the nitrite test solution, and negative pressure was applied. After 15 minutes of negative pressure, the indicator solution changed color 790, thereby demonstrating that interaction between the activator layer and the nitric oxide layer can produce nitric oxide, even while under negative pressure.

As will be understood by one of skill in the art, negative pressure may be applied to any of the nitric oxide delivering dressings disclosed herein, such as the dressings described in Figures 4-5 and elsewhere in the specification. A dressing, such as the dressings described in Figures 2A-2D may be placed over an activator layer and nitric oxide source layer which are placed in a wound, thereby delivering nitric oxide to a wound while simultaneously applying negative pressure wound therapy.

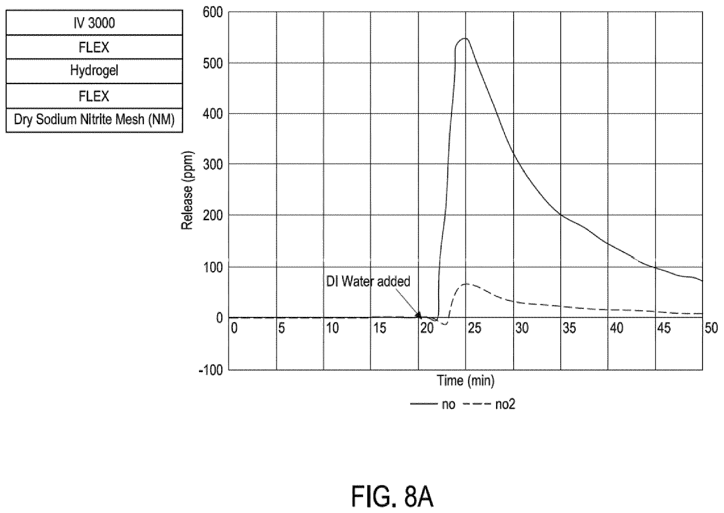

Figures 8A through 8C show examples of chemiluminescence experimental runs using a protocol similar to that described above. As will be understood by one of skill in the art, these measurements taken in these experimental runs are merely exemplary and the disclosures herein are not limited to such values. Figure 8A shows the experimental results when testing a dry sodium nitrate mesh embodiment with the arrangement shown in Figure 8A, including a polyurethane cover layer overlying a stretch polyester ADL layer, positioned over a hydrogel activator layer sandwiched between another stretch polyester ADL layer over a dry sodium nitrite mesh as shown in the figure. In this experimental run, after the DI water was added, the dry sodium nitrate mesh released approximately 550 ppm NO and 75 ppm NO₂ at its peak at the 25 minute mark, slowly reducing in concentration to approximately 80 ppm NO and 10 ppm NO₂ at the 50 minute mark.

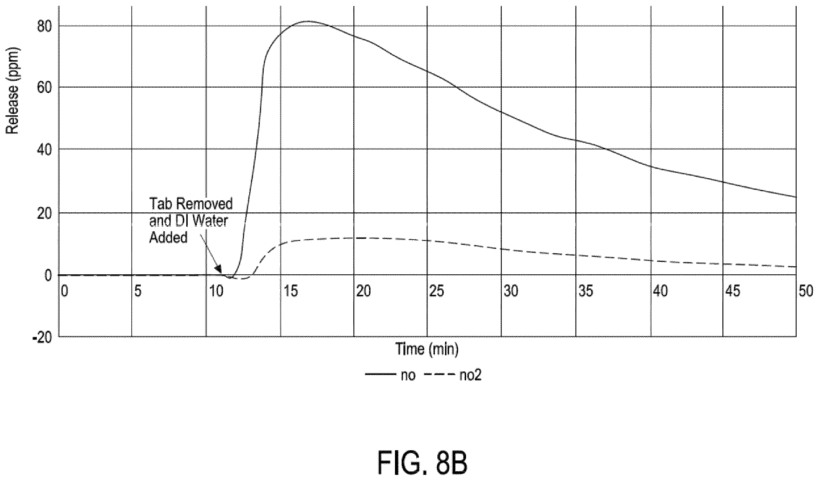

Figure 8B shows the experimental results when testing a full dressing design with a pull-out tab and self-sealing borders. The pull out tab is used to initially separate the nitric oxide source layer from the activator layer, therefore when the tab is removed and the dressing becomes wet, the interaction between the nitric oxide source layer and the activator layer produces nitric oxide. In this experimental run, after the DI water was added, the full dressing design with the pull-out tab and self-sealing borders released approximately 84 ppm NO and 15 ppm NO₂ at its peak at the 17 minute mark, slowly reducing in concentration to approximately 25 ppm NO and 5 ppm NO₂ at the 50 minute mark.

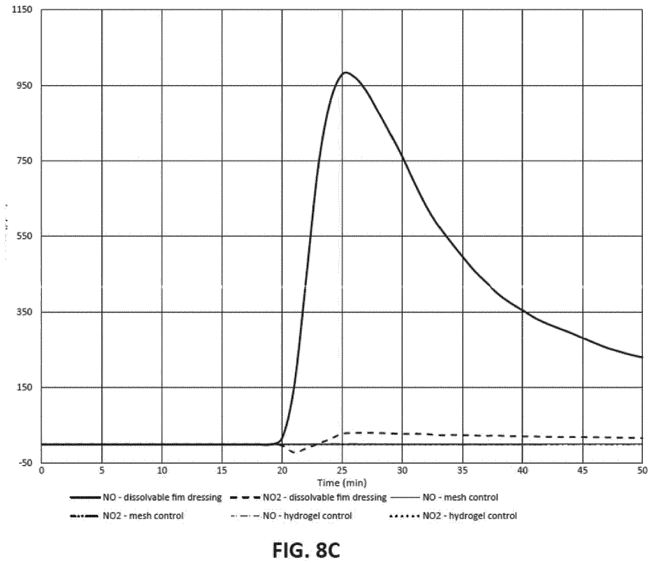

Figure 8C shows an example of the experimental results for a dressing containing a degradable film. Here, a degradable film was placed between the activator layer and the nitric oxide source layer, thereby generating nitric oxide once the degradable layer breaks down. In this experimental run, after the DI water was added, the dressing containing a degradable film released approximately 1000 ppm NO and 45 ppm NO₂ at its peak at the 25 minute mark, slowly reducing in concentration to approximately 225 ppm NO and 20 ppm NO₂ at the 50 minute mark. The experimental protocol was also utilized to test an activator layer containing sodium isoascorbate. In this experimental run, after the DI water was added, the activator layer containing sodium isoascorbate released approximately 52 ppm NO and 4 ppm NO₂ at its first peak at the 80 minute mark, 66 ppm NO and 5 ppm NO₂ at its second and maximum peak at the 110 minute mark slowly reducing in concentration to approximately 45 ppm NO and 2 ppm NO₂ at the 160 minute mark.

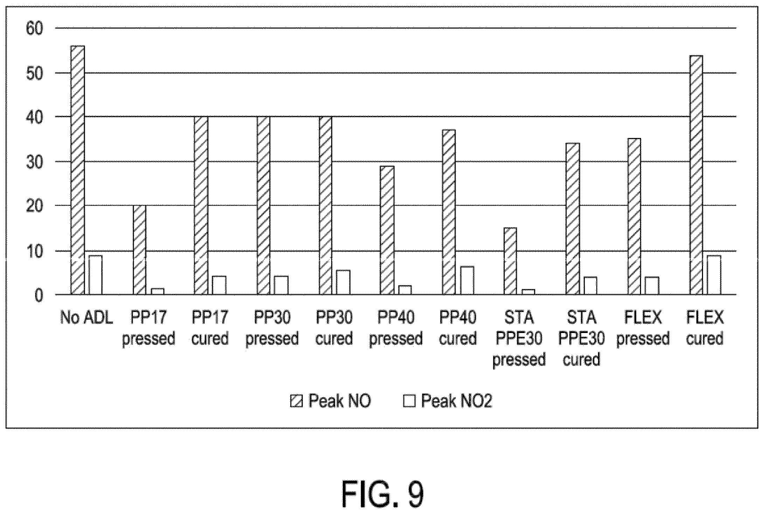

Figure 9 shows an example of the relative peak output in ppm for activator hydrogels (acid providing) either with an acquisition distribution layer or without, including polypropylene, polypropylethylene, or stretch polyester acquisition distribution layers with various gsm (g/m²). With no acquisition distribution layer, the peak NO and NO₂ concentrations were approximately 55 ppm and 10 ppm respectively; however, one of skill in the art will understand that an acquisition distribution layer may allow for improved fluid distribution and handling throughout a larger area such as a dressing. With a 17 gsm polypropylene pressed acquisition distribution layer, the peak NO and NO₂ concentrations were approximately 20 ppm and 2 ppm respectively. With a 17 gsm polypropylene cured acquisition distribution layer, the peak NO and NO₂ concentrations were approximately 40 ppm and 5 ppm respectively. As explained above, curing the acquisition distribution layer may allow for increased fluid transport and an increased rate of nitric oxide formation. With a polypropylene 30 g/m² pressed acquisition distribution layer, the peak NO and NO₂ concentrations were approximately 40 ppm and 5 ppm respectively. With a polypropylene 30 g/m² acquisition distribution layer, the peak NO and NO₂ concentrations were approximately 40 ppm and 5 ppm respectively. With a polypropylene 40 g/m² pressed acquisition distribution layer, the peak NO and NO₂ concentrations were approximately 30 ppm and 2 ppm respectively. With a polypropylene 40 g/m² cured acquisition distribution layer, the peak NO and NO₂ concentrations were approximately 38 ppm and 5 ppm respectively. With a polypropylethylene 30 g/m² pressed acquisition distribution layer, the peak NO and NO₂ concentrations were approximately 35 ppm and 3 ppm respectively. With a polypropylethylene 30 g/m² cured acquisition distribution layer, the peak NO and NO₂ concentrations were approximately 35 ppm and 3 ppm respectively. With a stretch polyester pressed acquisition distribution layer, the peak NO and NO₂ concentrations were approximately 35 ppm and 3 ppm respectively. With a FLEX pressed acquisition distribution layer, the peak NO and NO₂ concentrations were approximately 55 ppm and 8 ppm respectively.

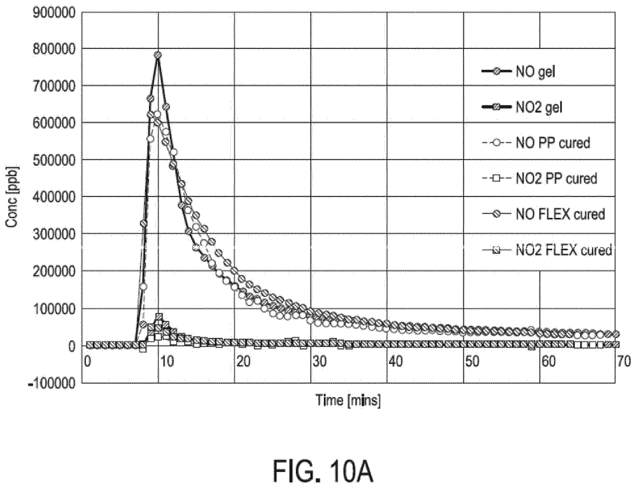

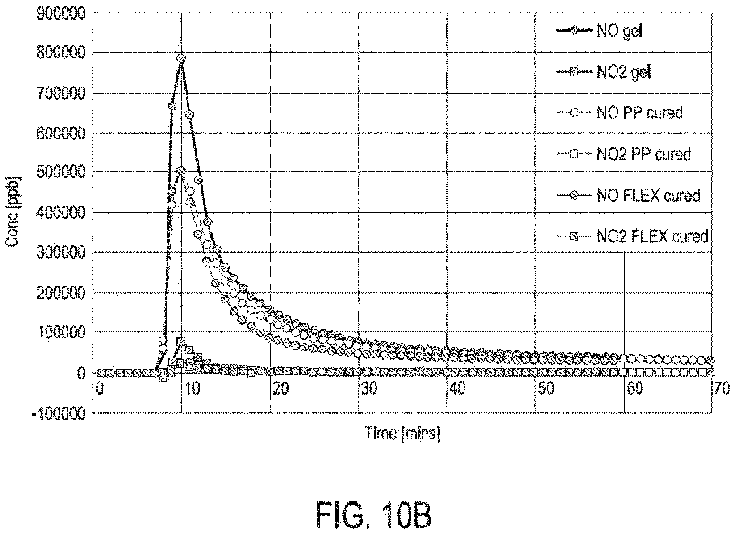

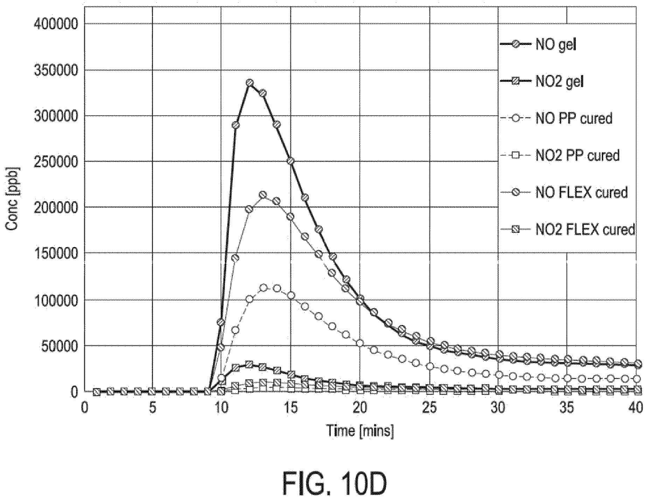

Figures 10A through 10D show examples of the concentration of NO and NO₂ over time for several embodiments incorporating an activator layer and nitric oxide providing layer. As shown in Figures 10A-10B, an activator layer containing approximately 2-3% sodium isoascorbate was tested with or without different acquisition distribution layers that were pressed or cured. The gel with no acquisition distribution layer produced (p indicating peak) pNO = 785ppm and pNO2 = 78ppm. The activator layer with a stretch polyester pressed into the gel produced pNO = 506ppm and pNO2 = 24ppm. For stretch polyester cured on the activator layer, pNO = 625ppm; pNO2 = 50ppm. For polypropylene pressed into the gel, the pNO = 508ppm and pNO2 = 26ppm. For polypropylene cured into the gel, the pNO = 624ppm and pNO2 = 26ppm.

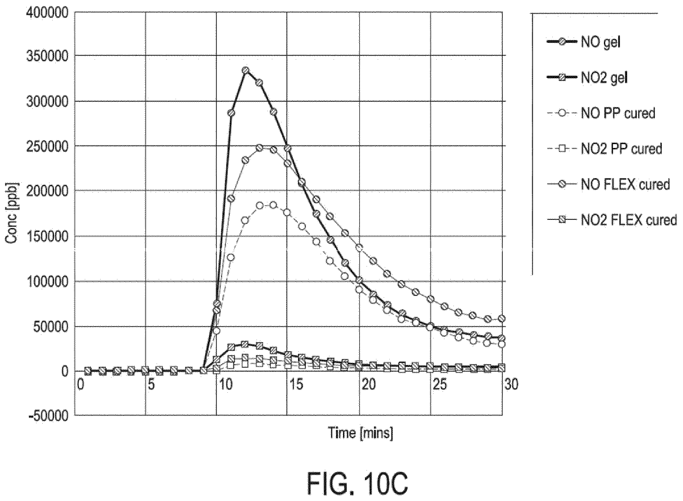

Figures 10C-10D show examples of the concentration of NO and NO₂ over time for an activator layer containing approximately 1-2% sodium isoascorbate with or without different acquisition distribution layers that were pressed or cured. The activator layer with no ADL produced pNO = 334ppm; pNO2 = 40ppm. For the stretch polyester acquisition distribution layer pressed into the activator layer, pNO = 211ppm and pNO2 = 10ppm. For the stretch polyester acquisition distribution layer cured into the activator layer, pNO = 247ppm and pNO2 = 14ppm. For the polypropylene acquisition distribution layer pressed into the activator layer, pNO = 112ppm and pNO2 = 5ppm. For the polypropylene acquisition distribution layer cured into the activator layer, pNO = 184ppm and pNO2 = 8ppm. As explained elsewhere in the specification, curing an acquisition distribution layer into an activator layer may improve fluid handling and nitric oxide production relative to nitrogen dioxide production.

### Xerogels and Hydrogel construction

Reference may be made throughout the specification to xerogels. A xerogel may be formed from a gel by drying with unhindered shrinkage. As will be understood by one of skill in the art, a xerogel is a gel that has very low free water content, so low that minimal reaction to form nitric oxide will occur without the addition of further water and/or liquid. For example, a xerogel may be substantially free of water in the dry state. Drying may be completed by any suitable means known in the art (e.g., freeze drying).

In certain examples, hydrogels (which may subsequently become xerogels after drying) may be generated with or without glycerol, and may contain a standard amount or double, triple, or quaruple the required amount of crosslinker PEG diacrylate as needed. A 2-Acrylamido-2-methyl-1-propanesulfonic acid sodium salt solution may be present in the xerogel. Hydrogels and xerogels may be created by converting 2-acrylamido-2-methyl-1-propanesulphonic acid (SA), stabilised with MEHQ as supplied, to a sodium salt by dissolving into water, then neutralising with 50% NaOH to pH 7.0 with cooling from a 10C water bath to form a solution of the neutralised acid (NaAMPS). Hydrogels can contain about 5.393 wt% 2-acrylamido-2-methyl-1-propanesulphonic acid (equating to 1.0 SA), about 4.654 wt% 2-acrylamido-2-methyl-1-propanesulphonic acid (equating to 0.85 SA), about 2.839 wt% 2-acrylamido-2-methyl-1-propanesulphonic acid (equating to 0.5 SA), and/or a range of between about 1.457 wt% 2-acrylamido-2-methyl-1-propanesulphonic acid (equating to 0.25 SA) to about 7.704 wt% 2-acrylamido-2-methyl-1-propanesulphonic acid (equating to 1.5 SA). Hydrogel prepolymers may be prepared by predispersing 2-hydroxy-2-methylpropiophenone photoinitiator into PEG diacrylate under minimal light, then mixing for 10-20 mins with a mixture of 58% aqueous sodium 2-acrylamido-2-methyl-1-propanesulfonate solution (Na AMPS), (Sodium iso-ascorbate, pre-ground 2-Acrylamido-2-methyl-1-propanesulfonic acid (AMPS acid) and glycerol. The AMPS acid may be fully dissolved in the stirred Na AMPS solution prior to slowly adding the glycerol, and then the photoinitiator/diacrylate mixture in a water bath. In certain embodiments, hydrogels may also prepared with twice the normal amount of photoinitiator/crosslinker and/or the omission of glycerol and/or using triple the amounts of prepolymer mix in the moulds to form gels with three times the thickness.

### Nitric Oxide Generating Dressings Utilizing Sodium Nitrite

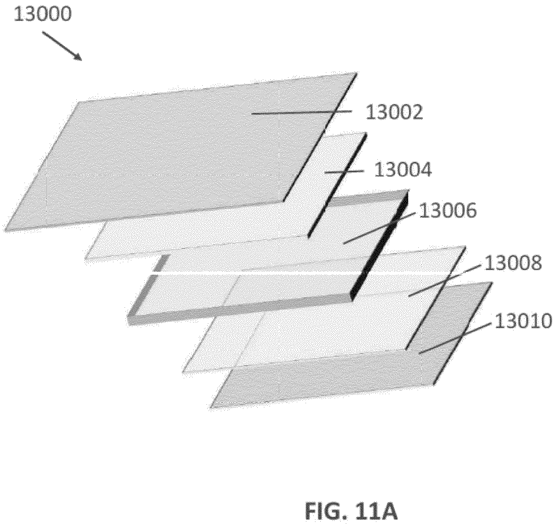

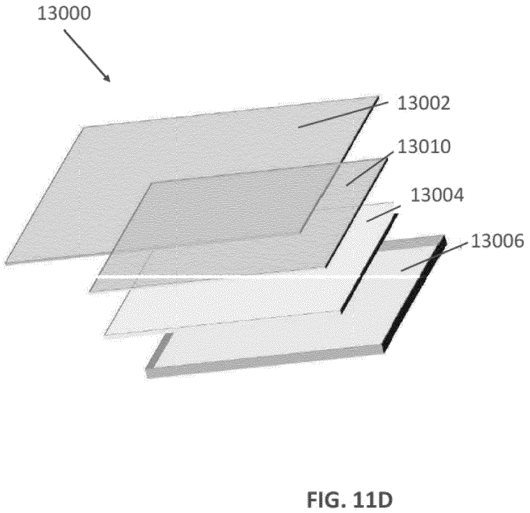

Figures 11A-11D depict embodiments of a nitric oxide generating wound dressing with various arrangements of layers. As will be understood by one of skill in the art, the nitrite providing layers disclosed herein may be prepared and provided separate from the wound dressing. For example, the nitrite providing layer may be applied to the wound and the wound dressing may be applied above the nitrite providing layer. One of skill in the art will further understand that the various layers depicted in Figures 11A-11D may be ordered in any suitable order and the orders depicted in the figures are merely examples. In some embodiments, the uppermost layer may be a cover layer 13002, which may have any of the same features, materials, or other details of cover layers disclosed herein, such as being constructed from a film. Said cover layer 13002 may be suitable for sealing a dressing over a wound and for connecting to a source of negative pressure and/or for maintaining negative pressure at a wound site. In certain embodiments, the border region of the cover layer 13002 may be attached to the skin around the wound, forming a seal, such that the wound exudate can be contained within the wound dressing 13000. Below the cover layer, there may be a masking or obscuring layer 13004 (heretofore referred to as a "masking layer") to prevent or limit visualization of the wound or the wound exudate through the cover layer 13002. The masking layer 13004 may be positioned beneath at least a portion of the cover layer 13002. In some embodiments, the masking layer 13004 can have any of the same features, materials, or other details of any of the other embodiments of the masking layers disclosed herein, including but not limited to having any viewing windows or holes. Examples of wound dressings with obscuring layers and viewing windows are described in International Patent Publications WO2013/007973 and WO2014/020440. Additionally, the masking layer 13004 may be positioned adjacent to the cover layer, or can be positioned adjacent to any other dressing layer as desired. In the illustrated embodiment, the masking layer 13004 is positioned between the cover layer 13002 and the activator layer 13006. As explained elsewhere herein and as will be understood by one of skill in the art, the activator layer may be an acid providing layer or other suitable layer. In certain embodiments, the masking layer 13004 can be adhered to or integrally formed with the cover layer 13002. The masking layer 13004 may be configured to have approximately the same size and shape as the activator layer 13006 so as to overlay it. The masking layer 13004 may be of a smaller area than the cover layer 13002. In certain embodiments, the masking layer 13004 can horizontally wick fluid and may function as an acquisition distribution layer as well.

In particular embodiments, the activator layer 13006 may have any of the same features, materials, or other details of any of the other embodiments of activator layers disclosed herein. For example, the activator layer 13006 may be adhesive and may be constructed of a hydrogel or xerogel configured to have a plurality of acidic groups or moieties which may provide protons in an aqueous environment. As explained elsewhere in the specification, under such acidic conditions nitrite ions from a nitric oxide source layer 13010 may be reduced to nitric oxide for delivery to a wound or intact skin. As explained elsewhere herein and as will be understood by one of skill in the art, the activator layer may also be a nitrite providing layer or other suitable layer. The activator layer 13006 (e.g. hydrogel layer) may include a plurality of perforations that extend through the thickness of the activator layer, as described elsewhere herein. The plurality of perforations may allow or facilitate passage of wound exudate through the activator layer, such that wound exudate below or around the activator layer can be transported to one or more additional absorbing layers and/or an evaporative layer or layers (e.g. cover layer) above the activator layer, thus preventing excessive buildup of wound exudate below the activator layer 13006. Additionally, the plurality of perforations may provide increased surface area of the activator layer, thereby increasing the absorption rate of the activator layer.

As shown in Figure 11A, in embodiments, an acquisition distribution layer 13008 may be placed between the activator layer 13006 and the nitrite providing layer 13010. In certain embodiments, the acquisition distribution layer 13008 may be constructed so as to advantageously horizontally wick fluid, such as wound exudate, as it is absorbed through the layers of the dressing 13000. Such lateral wicking of fluid may allow maximum distribution of the fluid through the activator layer 13006, enabling the activator layer 13006 to reach its full holding capacity. Further, acquisition distribution layer 13008 may facilitate nitric oxide production, as nitrite ion dissolved in fluid may spread across the surface of the activator layer 13006 more quickly. Some embodiments of the acquisition distribution layer 13008 may comprise viscose, polyester, polypropylene, cellulose, or a combination of some or all of these, and the material may be needle-punched. Some embodiments of the acquisition distribution layer 13008 may comprise cellulose in the range of 40-160 gsm (or about 40 to about 160 gsm), for example 80 (or about 80) gsm. Some embodiments of the acquisition distribution layer 13008 may comprise polyethylene in the range of 40-150 grams per square meter (gsm). In some embodiments, the acquisition distribution layer 13008 may have a thickness of 1.2 mm or about 1.2 mm, or may have a thickness in the range of about 0.5 mm to 3.0 mm, about 0.5 mm to about 3.0 mm., 0.7 mm to 2.5 mm, 0.9 mm to 2.1 mm, or 1.1 mm to 1.5 mm. In certain embodiments, the acquisition distribution layer 13008 may be constructed from a material which resists compression under the levels of negative pressure commonly applied during negative pressure therapy.

The acquisition distribution layer 13008 may include a plurality of loosely packed fibers, which may be arranged in a substantially horizontal fibrous network. In some embodiments, the acquisition distribution layer 13008 may consist of a mix of two fiber types. One may be a flat fiber which may be 20 µm to 50 µm in width, or approximately 20 µm to approximately 50 µm in width, and may comprise a cellulosic based material. The other fiber may be a two component fiber that has an inner core that is 8 µm to 10 µm in diameter, approximately is 8 µm to approximately 10 µm in diameter, 7 µm to 11 µm in diameter, 6 µm to 12 µm in diameter, or 5 µm to 13 µm in diameter and an outer layer with a thickness of 1 µm to 2 µm, approximately 1 µm to approximately 2 µm, 1 µm to 2.3 µm, 0.8 µm to 2.5 µm, or 0.5 µm to 3 µm. The two component fiber may be a mix of a polyethylene (PE) type material, and polyethylene terephthalate (PET). In some embodiments the inner core of the two component fiber may be PET and the outer layer may be PE. The PE/PET fibers may have a smooth surface morphology, while the cellulosic fibers may have a relatively rougher surface morphology. In some embodiments the ADL material may comprise about 60% to about 90% cellulosic fibers, for example approximately 75% cellulosic fibers, and may comprise about 10% to about 40% PE/PET fibers, for example approximately 25% PE/PET fibers. In some embodiments, the acquisition distribution layer 13004 may include split microfibers.

A majority of the fiber volume may extend horizontally (that is, parallel to the plane of the top and bottom surfaces of the material), or substantially or generally horizontally. In another embodiment, 80%-90% (or approximately 80% to approximately 90%) or more of the fiber volume may extend horizontally, or substantially or generally horizontally. In another embodiment, all or substantially all of the fiber volume may extend horizontally, or substantially or generally horizontally. In some embodiments, a majority, 80%-90% (or approximately 80% to approximately 90%) of the fibers or more, or even all or substantially all of the fibers, span a distance perpendicular to the thickness of the acquisition distribution layer 13004 (a horizontal or lateral distance) that is greater than the thickness of the acquisition distribution layer 13004. In some embodiments, the horizontal or lateral distance spanned by such fibers is 2 times (or about 2 times) or more, 3 times (or about 3 times) or more, 4 times (or about 4 times) or more, 5 times (or about 5 times) or more, or 10 times (or about 10 times) or more the thickness of the acquisition distribution layer 13008. The orientation of such fibers may promote lateral wicking of fluid through the acquisition distribution layer 13008. This may more evenly distribute fluid such as wound exudate throughout the acquisition distribution layer 13008. In some embodiments, the ratio of the amount of fluid wicked laterally across the acquisition distribution layer 13008 to the amount of fluid wicked vertically through the acquisition distribution layer 13008 under negative pressure may be 2:1 or more, or approximately 2:1 or more, or may be up to 10:1 or more, or approximately 10:1 or more, in some embodiments.

Continuing with Figure 11A, in embodiments a nitric oxide source layer 13010 may be provided beneath the acquisition distribution layer 13004. Such a nitric oxide source layer 13010 may have any of the same features, materials, or other details of any of the other embodiments of nitric oxide source layers disclosed herein, for instance, the nitric oxide source layer 13010 may be a nitrite providing layer. For example, the nitric oxide source layer may be a wet mesh imbued with sodium nitrite solution. In some embodiments, the nitric oxide source layer 13010 may be dry and include a dry nitrite source, such as dry sodium nitrite. Such dry sodium nitrite may be loaded into a material layer, said material layer constructed from a suitable material such as any material disclosed herein. As will be understood by one of skill in the art, a dry material and/or substance is one that is free or relatively free of liquid. For example, polypropylene, polyethylene, or melt extrudable fibers may be suitable materials for such a layer. In embodiments, such a nitric oxide source layer 13010 layer may need to be separated initially from the activator layer 13006 when the activator layer is a hydrogel so as to avoid reaction and production of nitric oxide prior to application to a wound and/or skin. As depicted in Figure 11A a dry fluid acquisition layer 13008 may serve to separate the nitric oxide source layer 13010 and a hydrogel activator layer 13004 prior to application. However, such a dry sodium nitrite providing layer may be adjacent to a xerogel activator layer 13006, since a xerogel will not be wet. In the instance of a xerogel, activation may occur upon contact with fluid such as wound exudate as the wound exudate wicks through the dressing. In the instance of a hydrogel, once fluid such as wound exudate comes into contact with the acquisition distribution layer 13008, then nitrite ions may come into contact with the acidic environment created by the activator layer, thereby generating nitric oxide which may then migrate into the wound and/or skin. In some embodiments, each of the layers such as the nitric oxide source layer, the activator layer, and any other suitable layer may be stored dry prior to use. Prior to application to the skin or wound, the layers may be wet by a suitable liquid such as saline.

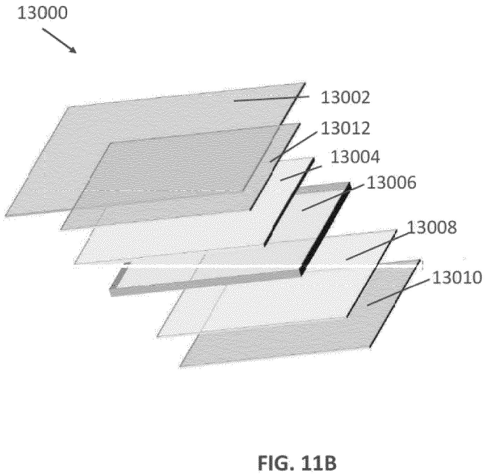

As depicted in Figure 11B, to maintain nitric oxide release there could be a number of layers containing dry sodium nitrite, for example a first nitric oxide source layer 13010 and a second nitric oxide source layer 13012 that would be 'activated' as wound fluid reaches and wets out the layer(s), enabling the sodium nitrite to come into contact with the acidic groups of the hydrogel or xerogel of activator layer 13006, thereby producing nitric oxide. In certain embodiments, there may be 2, 3, 4, 5, 6 or more layers containing dry sodium nitrite. As shown in Figure 11B, the masking layer 13004 may serve to prevent contact between the second nitric oxide source layer 13012 and the activator layer 13004. In certain embodiments, additional acquisition distribution layers and/or masking layers may be sandwiched with activator layers to provide additional sources of nitric oxide.

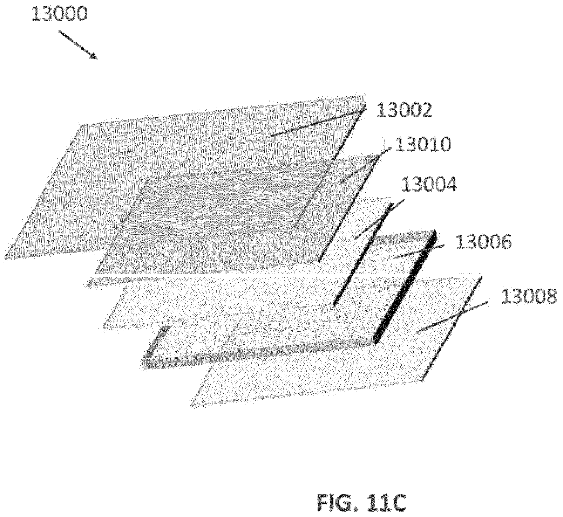

As depicted in Figures 11C-11D, in embodiments the activator layer 13006 may be positioned beneath the nitric oxide source layer, thereby relying on the dressing wetting out (such as from wound exudate) and activating the nitrite providing layer 13010.

### Nitric Oxide Dressings including ADLs

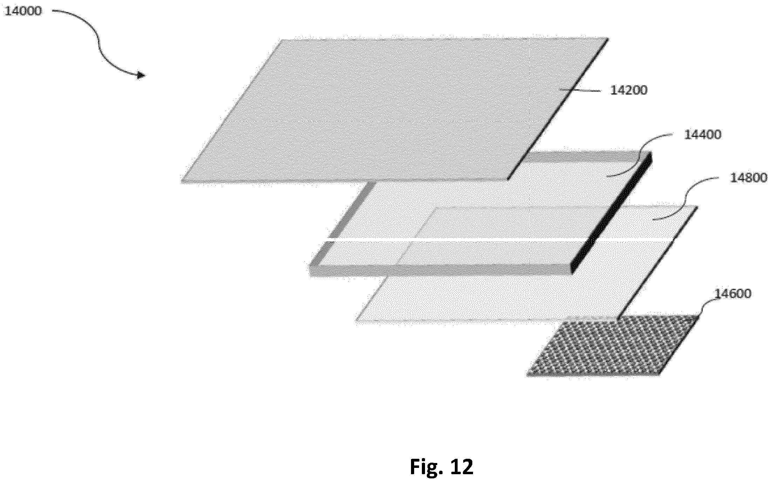

FIGS. 12-13 illustrate a wound dressing 14000 having nitric oxide generating layers. The wound dressing 14000 may be similar to the wound dressing 12000. The wound dressing 14000 may include a cover layer 14200, an activator layer or acid providing layer 14400, and a nitric oxide source layer or nitrite providing layer 14600, each of which can be similar to the cover layer 12200, the activator layer or acid providing layer 12400, and the nitric oxide source layer or nitrite providing layer 12600, respectively. As will be understood by one of skill in the art, the nitrite providing layer(s) 12600 disclosed herein may be prepared and provided separate from the wound dressing. For example, the nitrite providing layer 12600 may be applied to the wound and the wound dressing may be applied above the nitrite providing layer.

The cover layer 14200 may be similar to the cover layer 12200. The cover layer 14200 may have a greater length and width than other layers 14400, 14600, 14800, such that the cover layer 14200 defines a border region extending between outer perimeters of other layers and the outer perimeter of the cover layer 14200. The border region of the cover layer 14200 may be attached to the skin around the wound, forming a seal, such that the wound exudate can be contained within the wound dressing 14000.

In the illustrated embodiment, the wound dressing 14000 further includes an acquisition distribution layer (ADL) 14800. The acquisition distribution layer 14800 may be constructed so as to advantageously horizontally wick fluid, such as wound exudate, as it is absorbed through the layers of the dressing 14000. Such lateral wicking of fluid may allow maximum distribution of the fluid through the acid providing layer 14400, enabling the acid providing layer 14400 to reach its full holding capacity. Further, acquisition distribution layer 14800 may facilitate nitric oxide production, as nitrite ions dissolved in fluid may spread across the surface of the acid providing layer 14400 more quickly. Some embodiments of the acquisition distribution layer 14800 may comprise viscose, polyester, polypropylene, cellulose, or a combination of some or all of these, and the material may be needle-punched. Some embodiments of the acquisition distribution layer 14800 may comprise cellulose in the range of 1-220 grams per square meter (gsm) (or about 1 to about 220 gsm), 3-200 grams per square meter (gsm) (or about 3 to about 200 gsm), 5-190 gsm (or about 5 to about 190 gsm), 10-180 gsm (or about 10 to about 180 gsm), 20-170 gsm (or about 20 to about 170 gsm), or 40-160 gsm (or about 40 to about 160 gsm), for example 80 (or about 80) gsm. Some embodiments of the acquisition distribution layer 14800 may comprise polyethylene in the range of 3-200 gsm (or about 3 to about 200 gsm), 5-190 gsm (or about 5 to about 190 gsm), 10-180 gsm (or about 10 to about 180 gsm), 20-170 gsm (or about 20 to about 170 gsm), or 40-150 gsm. In some embodiments, the acquisition distribution layer 14800 may have a thickness of 1.2 mm or about 1.2 mm, or may have a thickness in the range of 0.07 mm to 7.0 mm, 0.1 mm to 5.0 mm, 0.5 mm to 3.0 mm, 0.7 mm to 2.5 mm, 0.9 mm to 2.1 mm, or 1.1 mm to 1.5 mm. The acquisition distribution layer 14800 may be constructed from a material which resists compression under the levels of negative pressure commonly applied during negative pressure therapy.

The acquisition distribution layer 14800 may include a plurality of loosely packed fibers, which may be arranged in a substantially horizontal fibrous network. In some embodiments, the acquisition distribution layer 14800 may consist of a mix of two or more fiber types. One may be a flat fiber which may be 20 µm to 50 µm in width, or approximately 20 µm to approximately 50 µm in width, and may comprise a cellulosic based material. The other fiber may be a two component fiber that has an inner core that is 8 µm to 10 µm in diameter, approximately is 8 µm to approximately 10 µm in diameter, 7 µm to 11 µm in diameter, 6 µm to 12 µm in diameter, or 5 µm to 13 µm in diameter and an outer layer with a thickness of 1 µm to 2 µm, approximately 1 µm to approximately 2 µm, 1 µm to 2.3 µm, 0.8 µm to 2.5 µm, or 0.5 µm to 3 µm. The two component fiber may be a mix of a polyethylene (PE) type material, and polyethylene terephthalate (PET). In some embodiments the inner core of the two component fiber may be PET and the outer layer may be PE. The PE/PET fibers may have a smooth surface morphology, while the cellulosic fibers may have a relatively rougher surface morphology. In some embodiments the ADL material may comprise about 60% to about 90% cellulosic fibers, for example approximately 75% cellulosic fibers, and may comprise about 10% to about 40% PE/PET fibers, for example approximately 25% PE/PET fibers. In some embodiments, the acquisition distribution layer 14800 may include split microfibers.

A majority of the fiber volume may extend horizontally (that is, parallel to the plane of the top and bottom surfaces of the material), or substantially or generally horizontally. In another embodiment, 80%-90% (or approximately 80% to approximately 90%) or more of the fiber volume may extend horizontally, or substantially or generally horizontally. In another embodiment, all or substantially all of the fiber volume may extend horizontally, or substantially or generally horizontally. In some embodiments, a majority, 80%-90% (or approximately 80% to approximately 90%) of the fibers or more, or even all or substantially all of the fibers, span a distance perpendicular to the thickness of the acquisition distribution layer 14800 (a horizontal or lateral distance) that is greater than the thickness of the acquisition distribution layer 14800. In some embodiments, the horizontal or lateral distance spanned by such fibers is 2 times (or about 2 times) or more, 3 times (or about 3 times) or more, 4 times (or about 4 times) or more, 5 times (or about 5 times) or more, or 10 times (or about 10 times) or more the thickness of the acquisition distribution layer 14800. The orientation of such fibers may promote lateral wicking of fluid through the acquisition distribution layer 14800. This may more evenly distribute fluid such as wound exudate throughout the acquisition distribution layer 14800. In some embodiments, the ratio of the amount of fluid wicked laterally across the acquisition distribution layer 14800 to the amount of fluid wicked vertically through the acquisition distribution layer 14800 under negative pressure may be 2:1 or more, or approximately 2:1 or more, or may be up to 10:1 or more, or approximately 10:1 or more, in some embodiments.

In some embodiments, at least some of the fiber volume of the acquisition distribution layer 14800 may extend vertically (that is, perpendicular to the plane of the top and bottom surfaces of the material), or substantially or generally vertically. In some embodiments, more than 10%, more than 20%, more than 30 %, more than 40%, more than 50%, more than 60%, more than 70%< more than 80%, or more than 90% of the fiber volume may extend vertically, or substantially or generally vertically. The orientation of such fibers may promote vertical wicking of fluid through the acquisition distribution layer 14800. In some embodiments, the ratio of the amount of fluid wicked vertically across the acquisition distribution layer 14800 to the amount of fluid wicked laterally through the acquisition distribution layer 14800 under negative pressure may be 2:1 or more, or approximately 2:1 or more, or may be up to 10:1 or more, or approximately 10:1 or more, in some embodiments.

In some embodiments, the acquisition distribution layer 14800 may be positioned below the acid providing layer 14400 as shown in FIGS. 12-13. In some embodiments, the acquisition distribution layer 14800 may be positioned above the acid providing layer 14400.

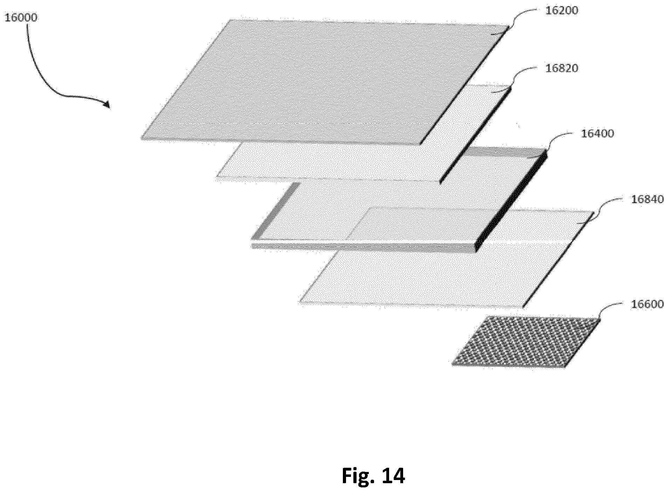

In some embodiments, a wound dressing having nitric oxide generating layers may include two or more acquisition distribution layers. FIGS. 14-15 illustrate a wound dressing 16000 having two acquisition distribution layers, a first acquisition distribution layer 16820 and a second acquisition distribution layer 16840. The wound dressing 16000 further includes a cover layer 16200, an acid providing layer 16400, and a nitrite providing layer 16600, which are similar with the cover layer 14200, the acid providing layer 14400, and the nitrite providing layer 14600, respectively. The first acquisition distribution layer 16820 and the second acquisition distribution layer 16840 are similar with the acquisition distribution layer 14800 of the wound dressing 14000.

As illustrated in FIGS. 14-15, the acid providing layer 16400 may be sandwiched between the first acquisition distribution layer 16820 and the second acquisition distribution layer 16840. In some embodiments, both of the first and second distribution layers may be positioned above the acid providing layer 16400 or below the acid providing layer 16400.

In some embodiments, one or more layers of a nitric oxide generating wound dressing can be transparent. For example, a cover layer, such as the cover layer 12200, the cover layer 14200 and the cover layer 16200, may be transparent. Further, an acid providing layer, such as the acid providing layer 12400, the acid providing layer 14400, and the acid providing layer 16400, can be translucent when the hydrogel material becomes wet, for example from contacting wound exudate. In some embodiments, a masking layer may be positioned within the wound dressing to prevent visualization of the wound or the wound exudate through the cover layer or the acid providing layer.

### Material Placement inside Hydrogel

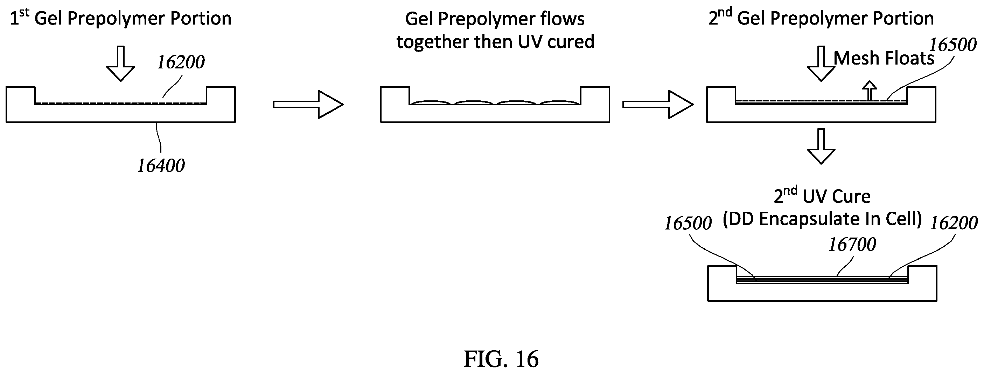

In some embodiments, the material layers may be physically implanted or immobilized to the hydrogel layer during the formation and/or curing of the hydrogel layer. FIG. 16 illustrates a process to physically implant or adhere a material layer within or onto a hydrogel layer during the formation of the hydrogel layer according to some embodiments. As illustrated in FIG. 16, a material layer 16200 may be positioned at a mold 16400 for curing a hydrogel layer, for example, at a bottom of the mold 16400. Before being positioned at the mold 16400, the material layer 16200 may be pretreated, for example with a wetting agent to make it hydrophilic, such that the affinity with hydrogel prepolymer is increased.

After the material layer 16200 is positioned at the bottom of the mold 16400, a first portion of a hydrogel prepolymer may be added. When the first portion of the hydrogel prepolymer is added, the pretreated material layer 16200 may be substantially wetted out with the first portion of the hydrogel prepolymer. The pretreated material layer 16200 positioned at the bottom of the mold 16400 may further facilitate the lateral spread of the hydrogel prepolymer and cause the bottom of the mold 16400 to also become substantially wetted with a continuous layer of the first portion of the hydrogel prepolymer. After the first portion of the hydrogel prepolymer is added, the material layer 16200 may rise from the bottom of the mold 16400 to the top of the hydrogel prepolymer. In some embodiments, the material layer 16200 may rise to the top of the hydrogel prepolymer in 10 minutes or less, 7 minutes or less, 5 minutes or less, 4 minutes or less, 3 minutes or less, 2 minutes or less, 1 minute or less, or more than 10 minutes. After the material layer 16200 rises, the first portion of the hydrogel prepolymer may be cured to form a first hydrogel layer 16500, and the material layer 16200 may be fixed on the top of the first hydrogel layer 16500, thereby masking the top side of the cured hydrogel. The first portion of the hydrogel prepolymer may be UV cured from top side, bottom side, or both sides, or any other suitable methods known in the art, or any other suitable methods known in the art.

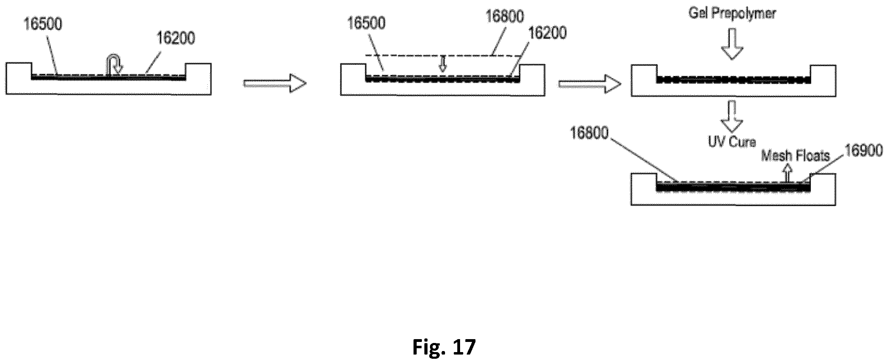

In some embodiments, after the first hydrogel layer 16500 is formed, a second portion of the hydrogel prepolymer may be added to the mold, over first hydrogel layer 16500 and the material layer 16200. After the second portion of the hydrogel prepolymer is added, the material layer 16200 may be encapsulated by the second portion of the hydrogel prepolymer and the first hydrogel layer 16500. The material layer 16200 may not rise or float, because it is immobilized to the first hydrogel layer 16500. Then the second portion of the hydrogel prepolymer may be cured to form a second hydrogel layer 16700, and the material layer 16200 may be encapsulated by the hydrogel layers 16500 and 16700 which may be integrated into a single layer. The material layer 16200 implanted embedded within the integrated hydrogel layer formed with hydrogel layers 16500 and 16700 may increase the structural integrity of the hydrogel layer. For example, when the hydrogel layer absorbs water, it may swell, and the material layer be act as a reinforcing layer preventing the hydrogel from stretching and falling apart. In some embodiments, the refractive index of the material layer and the hydrogel layer may be similar such that the material layer is completely invisible and the hydrogel layer appears as a single sheet of clear/transparent material. As will be understood by one of skill in the art and reiterated later in the specification, the aforementioned description of a method for addition of a material layer to a hydrogel is not limiting and may be performed in any suitable order and may involve the addition or removal of certain steps. FIG. 17 illustrates a process to physically implant a material layer onto both upper and lower sides of a hydrogel layer during the formation of the hydrogel layer according to some embodiments. However, one of skill in the art will understand that material layers may be added to one side only. As illustrated in FIG. 17, after the first hydrogel layer 16500 having the material layer 16200 is formed as described in relation to FIG. 16, it may be taken out from the mold 16400, flipped, and placed back into the mold 16400, such that the side of the hydrogel layer 16500 having the material layer 16200 faces the bottom of the mold 16400. Then, another material layer 16800 is positioned over the hydrogel layer 16500, and subsequently a second portion of the hydrogel prepolymer is added above the hydrogel layer 16500 and the material layer 16800. The material layer 16800 may float and rise to the top of the second portion of the hydrogel prepolymer in a similar manner to the material layer 16200 being floated during the formation of the hydrogel layer 16500 as described with regard to FIG. 16. After the material layer 16800 rises to the top of the second portion of the hydrogel prepolymer, the second portion of the hydrogel prepolymer may be cured to form a hydrogel layer 16900 with the hydrogel layer 16500, and the material layer 16800 may be fixed on the top of the hydrogel layer 16900, thereby masking the top side of the hydrogel layer 16900. The second portion of the hydrogel prepolymer may be cured by UV from top side, bottom side, or both sides, or any other suitable methods known in the art. As a result, the hydrogel layer 16900 may be sandwiched between the material layers 16200 and 16800, which are immobilized to the hydrogel layer 16900.

### Wound Dressings with Composite Layers

The activator layer, such as a gel layer disclosed herein, is integrated with another material to form a composite layer. In examples, the gel layer may be a hydrogel. Such integration may be produced through curing of a gel polymer (such as a hydrogel) or other suitable techniques. As will be described in greater detail below, for example, the gel may be integrated with an ADL layer such as any ADL layer described herein to form a composite acquisition distribution layer. In certain examples, the gels may be integrated with SLIMCORE, produced by Libeltex, or other non-woven acquisition distribution layers as described further herein. For example, the ADL may be an air bonded web multi-layered acquisition distribution layer. In certain examples, the ADLs may include hydrophilic PET and/or bi-component fibers. In further examples, the gel may be integrated with absorbent materials to form a composite absorbent layer as describer in greater detail below, including material such as fibrous gelling material such as cellulose fibers. For example, the cellulose/cellulose ethyl sulphonate fibres found in DURAFIBER, produced by Specialty Fibres and Materials Ltd and incorporated into products by Smith + Nephew. Further examples of absorbent materials include gelling fibers such as the hydrofibers in AQUACEL, produced by Convatec. Such integrated composite layers may serve to provide improved fluid handling, thereby directing nitrite into the composite structure for improved interaction with the activator layer, thereby generating and delivering nitric oxide. As will be understood by one of skill in the art, the nitrite providing layer(s) disclosed herein may be prepared and provided separate from the wound dressing. For example, the nitrite providing layer may be applied to the wound and the wound dressing may be applied above the nitrite providing layer.

In some examples, the gel layers and/or composite layers described herein may include an interleave within the gel layer. The interleave may be in the form of a thin mesh that is located within a layer of the gel for structural support and may be constructed from polypropylene or any suitable polymer disclosed herein. The interleave may be constructed from a porous, structural material which provides greater integrity to an otherwise poorly cohesive gel. The interleave may be located in the center of a gel slab to bring cohesion for thicker samples. However, one of skill in the art will understand that the interleave may be positioned in any suitable location within the gel, such as near the top or bottom. Further, the interleave may be configured so as not to impair the passage of fluid through the thickness of the gel.

### Composite Acquisition Distribution Layer

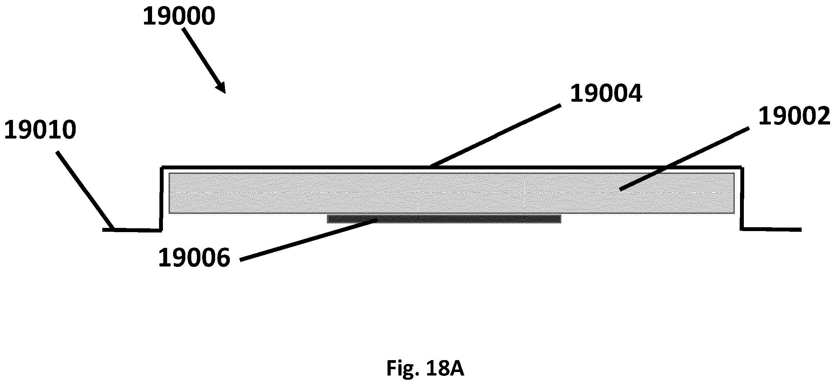

As explained above, in certain embodiments the activator layer, such as a hydrogel layer, may be embedded with an acquisition distribution layer to form a composite layer. Figure 18A depicts a wound dressing 19000 similar to the wound dressings depicted in Figures 11A-15; however, here wound dressing 19000 includes a composite acquisition distribution layer (ADL) 19002. The composite ADL may be formed by applying activator gel (including any suitable activator layer material described herein) in liquid form to an ADL (such as any suitable ADL described herein) or vice-versa. As described elsewhere in the specification, the activator gel may be a hydrogel or xerogel configured to have a plurality of acidic groups or moieties which may provide protons in an aqueous environment. In the case of a xerogel, an ADL may be embedded while dry within the xerogel. In the case of a hydrogel, the hydrogel may be applied in liquid and/or gel form. The activator hydrogel material may then be cured to form a composite ADL 19002. The activator layer may be cured by any suitable techniques, such as by applying energy to the system in the form of UV light, irradiation (such as gamma), or heat (optionally, in combination with the appropriate initiators such as a photo-initiator and/or a thermal initiator). Hardener chemical additives may also be utilized to cure. A cover layer 19004 (such as described herein), may be applied over the composite ADL and a nitrite providing layer 19006 may be provided below the composite ADL layer or above the composite ADL layer below the cover layer. The nitrite providing layer 19006 may be provided as part of a dressing or applied separately. Further, a wound contact layer (not shown) such as described elsewhere in the specification, may be positioned beneath the dressing and over a wound. As will be understood by one of skill in the art, the activator gel in liquid form may be applied in any suitable manner such that the ADL encompasses a bottom portion of the composite ADL layer, a top portion of the composite ADL layer, or a middle portion of the composite ADL layer. In embodiments, the ADL may encompass the entire dimensions of the composite ADL layer. In some examples, the ADL may be coated in a hydrophilic agent prior to addition of the activator layer in liquid gel form. In certain examples, the activator gel in liquid form may be cast directly on top of an ADL layer to form a composite ADL and/or the ADL may be pressed into the top of the activator layer prior to curing the activator layer into a gel. As will be understood by one of skill in the art, the activator gel material and ADL material may be merged together in any suitable arrangement.

In certain embodiments, the ADL may be made more rigid prior to casting into a composite ADL, via any suitable means such as coating or temperature treating. Further suitable methods may include bonding methods, such as heat bonding or adhesive bonding. Additional suitable methods may include thermal setting, increasing the thickness/grammage, increasing fiber stiffness/thickness, laminating and/or annealing. A more rigid ADL may serve to improve fluid uptake in the composite ADL by increasing surface area. Further methods for curing and forming the composite ADL layer will be described below.

The cover layer 19004 may be similar to the cover layers disclosed elsewhere herein. The cover layer 19004 may have a greater length and width than other layers such that the cover layer 19004 defines a border region 19010 extending between outer perimeters of other layers and the outer perimeter of the cover layer 19008. The border region of the cover layer 19010 may be attached to the skin around the wound, forming a seal, such that the wound exudate can be contained within the wound dressing 19000.

In certain embodiments, the composite ADL 19002 may be constructed so as to advantageously horizontally and/or vertically wick fluid, such as wound exudate, as it is absorbed into the wound dressing 19000. Such wicking of fluid may allow maximum distribution of the fluid through the composite ADL layer 19002, thereby enabling the composite ADL 19002 to reach full holding capacity or close to full capacity. Further, the composite ADL 19002 may facilitate nitric oxide production, as nitrite ions dissolved in fluid may spread across and throughout the composite ADL 19002 more quickly than in the activator gel alone. Some embodiments of the ADL within the composite ADL layer 19002 may comprise viscose, polyester, polypropylene, cellulose, or a combination of some or all of these, and the material may be needle-punched. Further, the composite ADL layer can include fibers such as polyethylene, polyamides, and/or suitable blends thereof. Additional suitable materials may include copolymers of any polymers disclosed herein and core/sheath fibers with more than one material per fiber. Any suitable ADL material disclosed herein may be used. Some embodiments of the ADL within the composite ADL layer 19002 may comprise cellulose in the range of 1-220 grams per square meter (gsm) (or about 1 to about 220 gsm), 3-200 grams per square meter (gsm) (or about 3 to about 200 gsm), 5-190 gsm (or about 5 to about 190 gsm), 10-180 gsm (or about 10 to about 180 gsm), 20-170 gsm (or about 20 to about 170 gsm), or 40-160 gsm (or about 40 to about 160 gsm), for example 80 (or about 80) gsm. Some embodiments of the composite acquisition distribution layer 19002 may comprise polyethylene in the range of 3-200 gsm (or about 3 to about 200 gsm), 5-190 gsm (or about 5 to about 190 gsm), 10-180 gsm (or about 10 to about 180 gsm), 20-170 gsm (or about 20 to about 170 gsm), or 40-150 gsm. In some embodiments, the ADL may have a thickness of 1.2 mm or about 1.2 mm, or may have a thickness in the range of 0.07 mm to 7.0 mm, 0.1 mm to 5.0 mm, 0.5 mm to 3.0 mm, 0.7 mm to 2.5 mm, 0.9 mm to 2.1 mm, or 1.1 mm to 1.5 mm. The ADL may be constructed from a material which resists compression under the levels of negative pressure commonly applied during negative pressure therapy, such as described herein.

The composite ADL 19002 may include a plurality of loosely packed fibers, which may be arranged in a substantially horizontal fibrous network. In some embodiments, the ADL within the composite ADL 19002 may consist of a mix of two or more fiber types. One may be a flat fiber which may be 20 µm to 50 µm in width, or approximately 20 µm to approximately 50 µm in width, and may comprise a cellulosic based material. In certain examples, the fiber geometries may include star-shaped, oval, or other suitable shapes. The other fiber may be a two component fiber that has an inner core that is 8 µm to 10 µm in diameter, approximately is 8 µm to approximately 10 µm in diameter, 7 µm to 11 µm in diameter, 6 µm to 12 µm in diameter, or 5 µm to 13 µm in diameter and an outer layer with a thickness of 1 µm to 2 µm, approximately 1 µm to approximately 2 µm, 1 µm to 2.3 µm, 0.8 µm to 2.5 µm, or 0.5 µm to 3 µm. The two component fiber may be a mix of a polyethylene (PE) type material, and polyethylene terephthalate (PET). In some embodiments, the inner core of the two component fiber may be PET and the outer layer may be PE. The PE/PET fibers may have a smooth surface morphology, while the cellulosic fibers may have a relatively rougher surface morphology. In some embodiments the ADL material may comprise about 60% to about 90% cellulosic fibers, for example approximately 75% cellulosic fibers, and may comprise about 10% to about 40% PE/PET fibers, for example approximately 25% PE/PET fibers. In some embodiments, the ADL may include split microfibers.

A majority of the fiber volume in the composite ADL may extend horizontally (that is, parallel to the plane of the top and bottom surfaces of the material), or substantially or generally horizontally. In another embodiment, 80%-90% (or approximately 80% to approximately 90%) or more of the fiber volume may extend horizontally, or substantially or generally horizontally. In embodiments, all or substantially all of the fiber volume may extend horizontally, or substantially or generally horizontally. In some embodiments, a majority, 80%-90% (or approximately 80% to approximately 90%) of the fibers or more, or even all or substantially all of the fibers, span a distance perpendicular to the thickness of the acquisition distribution layer (a horizontal or lateral distance) that is greater than the thickness of the composite acquisition distribution layer 19002. In some embodiments, the horizontal or lateral distance spanned by such fibers is 2 times (or about 2 times) or more, 3 times (or about 3 times) or more, 4 times (or about 4 times) or more, 5 times (or about 5 times) or more, or 10 times (or about 10 times) or more than the thickness of the composite ADL 19002. The orientation of such fibers may promote lateral wicking of fluid through the composite ADL, thereby more evenly distributing fluid such as wound exudate throughout the composite ADL 19002. In some embodiments, the ratio of the amount of fluid wicked laterally across the composite ADL 19002 to the amount of fluid wicked vertically through the composite ADL 19002 under negative pressure may be 2:1 or more, or approximately 2:1 or more, or may be up to 10:1 or more, or approximately 10:1 or more, in some embodiments.

In some embodiments, at least some of the fiber volume of the composite ADL 19002 may extend vertically (that is, perpendicular to the plane of the top and bottom surfaces of the material), or substantially or generally vertically. In certain embodiments, more than 10%, more than 20%, more than 30 %, more than 40%, more than 50%, more than 60%, more than 70%< more than 80%, or more than 90% of the fiber volume may extend vertically, or substantially or generally vertically. The orientation of such fibers may promote vertical wicking of fluid through the composite ADL 19002. In some embodiments, the ratio of the amount of fluid wicked vertically across the composite ADL 19002 to the amount of fluid wicked laterally through the composite ADL 19002 under negative pressure may be 2:1 or more, or approximately 2:1 or more, or may be up to 10:1 or more, or approximately 10:1 or more, in some embodiments.

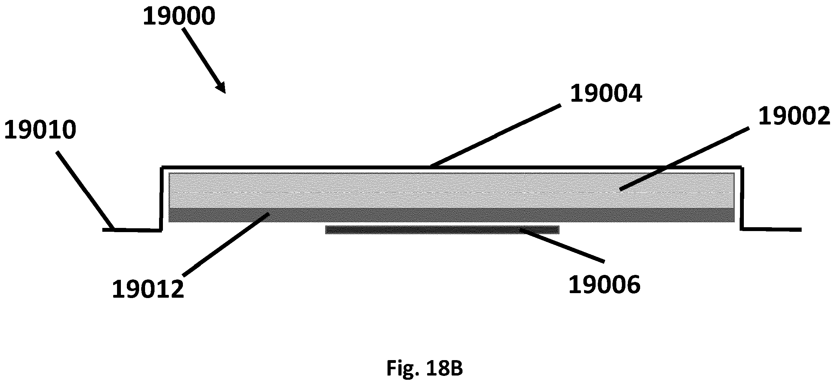

In some embodiments, as depicted in Figure 18B, another separate ADL 19012, such as described herein, may be placed below the composite ADL to further facilitate fluid wicking and nitrite distribution throughout the composite ADL. As will be understood by one of skill in the art, the ADL may instead be positioned over the composite ADL or multiple ADLs may be placed on the top and bottom of the composite ADL. One of skill in the art will also understand that the nitrite providing layer may be duplicated such that one or more nitrite providing layer may be positioned elsewhere in the dressing such as above the composite ADL. Multiple ADLs, composite ADLS, and/or nitrite providing layers may be suitably incorporated into a single dressing.

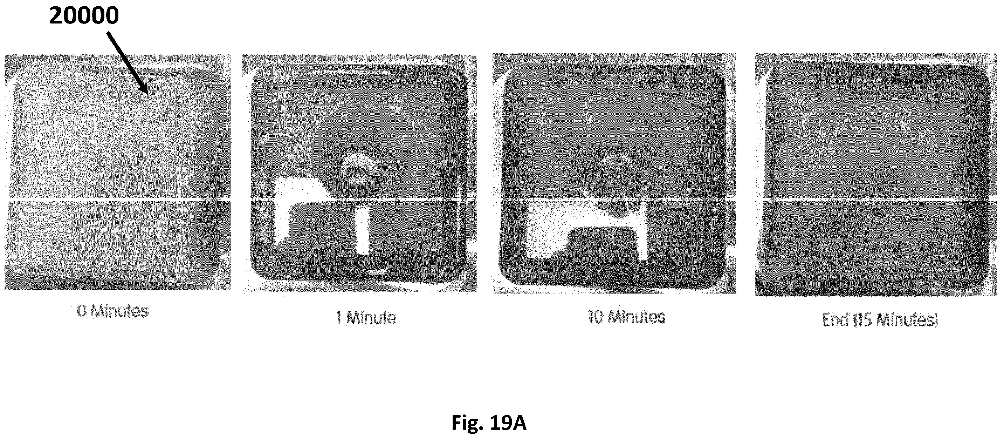

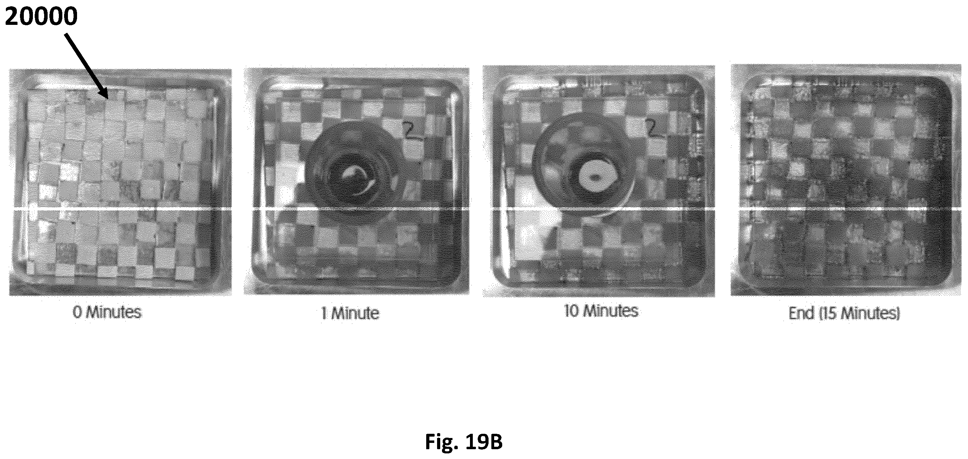

Figures 19A-19B depict fluid handling tests on a composite ADL 20000 such as the composite ADLs described above in related to Figures 18A-18B. Figure 19A depicts fluid wicking through the composite ADL 20000 as represented by the red liquid. As can be seen in the figure, after one minute, fluid has distributed across almost the entire dressing. Such fast fluid distribution will facilitate interaction between the hydrogen ions from the activator layer gel and the nitrite passing through the dressing. Figure 19B depicts a composite ADL layer 20000, similar to the layers described above in relation to Figures 18A-19A. Here, squares of the activator gel and squares of ADL were placed onto a sheet of material such as cover layer material as described herein. The ADL and activator gel squares were placed in an alternating checkerboard pattern to produce a checkerboard composite ADL. Fluid distribution across the composite ADL was observed at 1 minute.

### Composite Absorbent Layers

As explained above, in further examples, the gel may be integrated with absorbent materials to form a composite absorbent layer. Such a composite absorbent layer may include material such as fibrous gelling material such as cellulose fibers. For example, the cellulose/cellulose ethyl sulphonate fibers found in DURAFIBER, produced by Specialty Fibres and Materials Ltd and incorporated into products by Smith + Nephew. Further examples of absorbent materials include gelling fibers such as the hydrofibers in AQUACEL, produced by Convatec. In certain embodiments, the absorbent material may be foam or another suitable material.

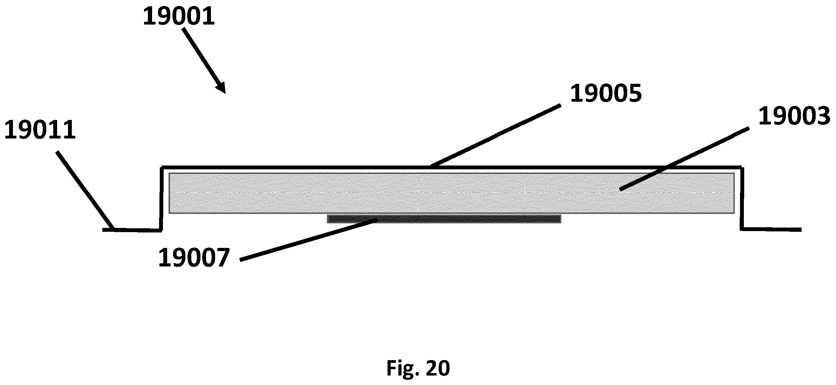

Figure 20 depicts a wound dressing 19001 similar to the wound dressings depicted in Figures 11A-15 and 18A-18B; however, here wound dressing 19001 includes a composite absorbent layer 19003. The composite absorbent layer may be formed by applying activator gel (including any suitable activator layer material described herein) in liquid form to an absorbent layer (such as any suitable absorbent described herein) or vice-versa. As described elsewhere in the specification, the activator gel may be a hydrogel or xerogel configured to have a plurality of acidic groups or moieties which may provide protons in an aqueous environment. In the case of a xerogel, an absorbent may be embedded while dry within the xerogel. In the case of a hydrogel, the hydrogel may be applied in liquid and/or gel form. The activator hydrogel material may then be cured to form a composite absorbent layer 19003. The activator layer may be cured by any suitable techniques, such as by applying energy to the system in the form of UV light, irradiation (such as gamma), or heat (optionally, in combination with the appropriate initiators such as a photo-initiator and/or a thermal initiator). Hardener chemical additives may also be utilized to cure. A cover layer 19005 (such as described herein), may be applied over the composite absorbent layer and a nitrite providing layer 19007 may be provided below the composite absorbent layer or above the composite ADL layer below the cover layer. The nitrite providing layer 19007 may be provided as part of a dressing or applied separately. Further, a wound contact layer (not shown) such as described elsewhere in the specification, may be positioned beneath the dressing and over a wound. As will be understood by one of skill in the art, the activator gel in liquid form may be applied in any suitable manner such that the absorbent encompasses a bottom portion of the composite absorbent layer, a top portion of the composite absorbent layer, or a middle portion of the composite absorbent layer. In embodiments, the absorbent may encompass the entire dimensions of the composite absorbent layer. In some examples, the absorbent may be coated in a hydrophilic agent prior to addition of the activator layer in liquid gel form. In certain examples, the activator gel in liquid form may be cast directly on top of an absorbent to form a composite absorbent layer and/or the absorbent may be pressed into the top of the activator layer prior to curing the activator layer into a gel. As will be understood by one of skill in the art, the activator gel material and absorbent material may be merged together in any suitable arrangement.

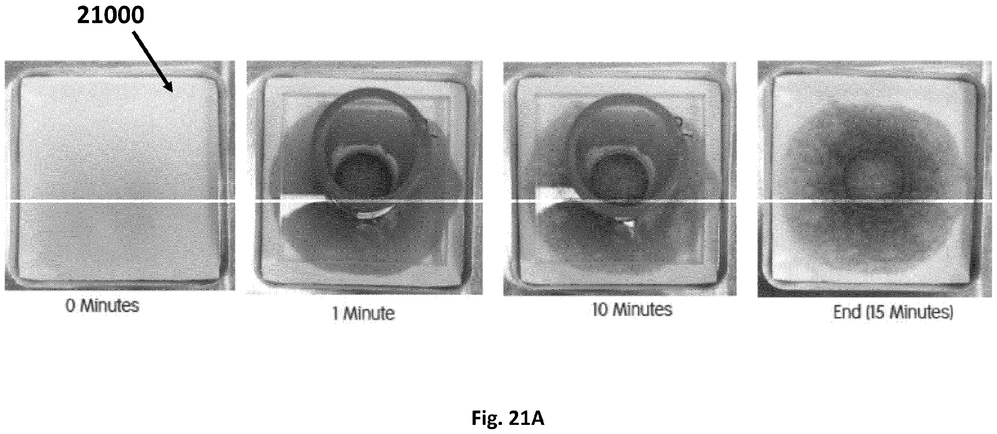

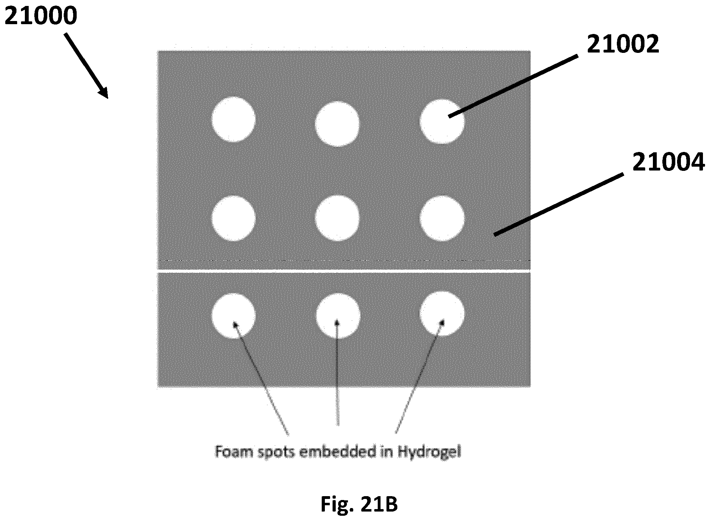

In some embodiments, foam may be used to form a composite absorbent layer (aka a composite foam layer) with an activator gel, similar to the embodiments described above in relation to Figures 18A-20. As will be understood by one of skill in the art, the composite foam layer may be incorporated into a wound dressing in any manner as described above in relation to the composite layers of Figures 18A-20. Further, the composite foam layer may be utilized in combination with other foam layers and nitrite providing layers similar to the additional ADL layers and one of more nitrite providing layers of Figures 18A-19B. In certain embodiments, before curing, the uncured activator gel material in liquid form may be poured into a foam and then cured in the foam to form a composite foam layer. Figure 21A depicts a fluid handling test with a composite foam layer 21000 where uncured activator layer material liquid has been poured into the foam and cured. As shown in the figure, the composite foam layer 21000 absorbed all fluid and the fluid distributed in a radius about the liquid source. Figure 21B depicts a composite foam layer included foam portions 21002 embedded in the activator gel 21004. As will be understood by one of skill in the art, the foam portions may penetrate through the entire thickness of the composite foam layer or only partially through the composite foam layer. The foam portions may be in a rounded shape, square shape, disc shape, or any suitable shape. The foam portions may be arranged in an array, as shown here or may be in a random pattern.

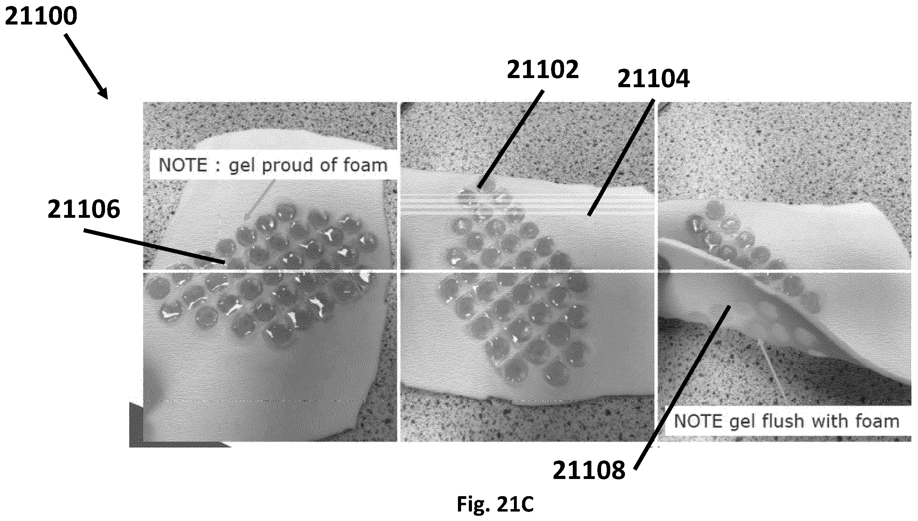

Figure 21C shows an embodiment of a composite foam layer similar to the composite foam layer of Figure 21B. Here, in contrast to Figure 21B, portions of activator gel material are cured into the foam layer. The activator gel 21102 may penetrate through the entire thickness of the foam 21104 or only partially through the foam. As will be understood by one of skill in the art, the activator gel 21102 may be arranged in an array, a random pattern, or any suitable arrangement. Further, the activator gel 21102 may be rounded or in another suitable shape. As depicted in Figure 21C, the activator gel may protrude from the foam layer 21106 or be flush 21108 with the foam layer. The activator gel may protrude from both sides or only one side or be flush on both sides or only one side. In certain embodiments, the activator gel is in the form of discs placed within punchouts in the foam.

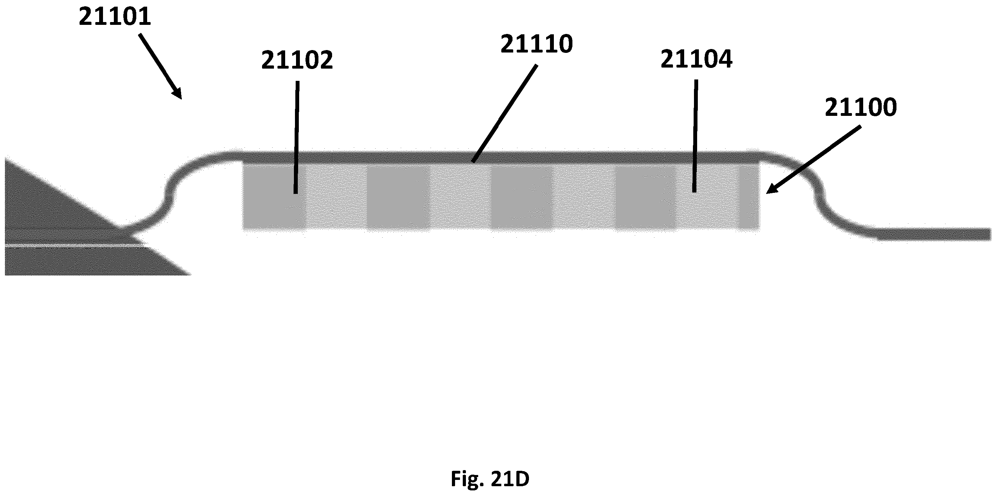

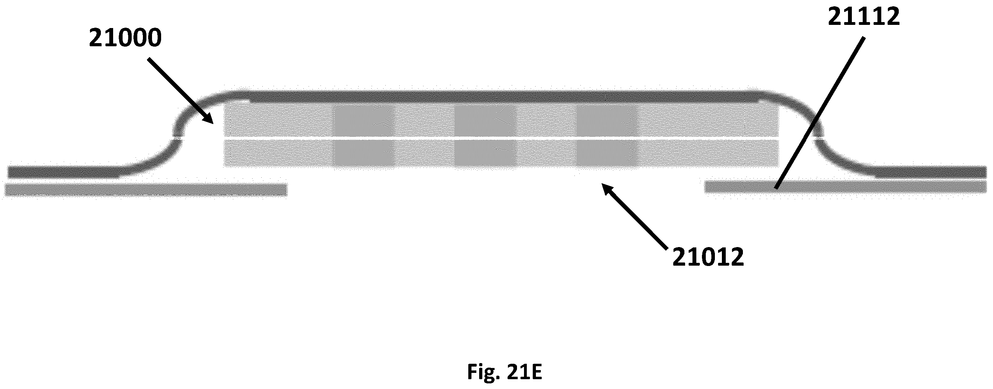

Figure 21D depicts a diagram of a side view of a wound dressing 21101, including the composite foam layer 21100 of Figure 21C. Here a cover layer 21110, similar to the other cover layers disclosed herein overlies the composite foam layer, which includes alternating activator layer gel portions 21102 and foam 21104. Figure 21E depicts the same composite foam layer, now with a wound contact layer 21112 underneath constructed from any of the materials described herein. In certain embodiments, the silicone wound contact layer may extend across the entire bottom of the wound dressing or may only extend partially across the underside of the wound dressing so as to form a window 21012. As will be understood by one of skill in the art, the wound contact layer may be constructed from any suitable material disclosed herein. For example, the wound contact layer may be constructed from silicone, skin-friendly pressure sensitive adhesive, gentle acrylic-based adhesive, polyurethane-based adhesive, hydrocolloid, or other suitable material. The window may allow for ease of delivery of nitric oxide to the wound. One of skill in the art will understand that a nitrite providing layer may be attached directly to the underside or topside of the composite foam layer and/or provided separately and placed beneath or above the composite foam layer.

In some embodiments, rather than foam or ADL, a composite layer may be formed with superabsorbent material. The superabsorbent material may be embedded within the composite superabsorber layer by any suitable means, such as in distinct patterns or arrays of superabsorber within an activator layer gel. In embodiments, the superabsorbent may be distributed throughout the composite absorbent layer such that particles of superabsorbent are distributed equally throughout or concentrated in certain regions of the composite absorbent layer. Such embodiments may serve to absorb and capture more fluid within the superabsorbent portions. In particular, embodiments, the composite super absorber layer may contain between about: 10 to 90% superabsorbent, such as 20 to 80%, 30 to 70%, 40 to 60%, or about 50% superabsorbent. In one example, the gel may be removed entirely to simply have a layer of superabsorbent with embedded acid providing moieties.

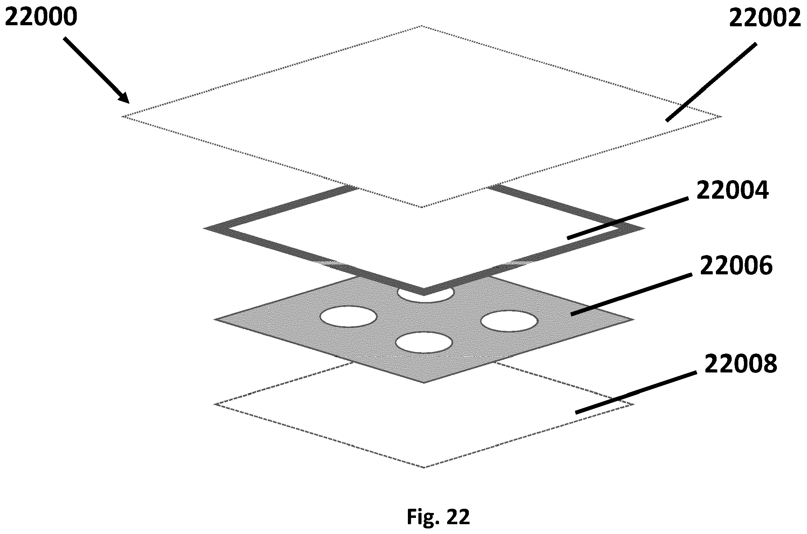

Figure 22 depicts a wound dressing 22000 with a cover layer 22002, an absorbent layer that may be foam 22004, a perforated activator gel layer 22006, and a nitrite providing layer 22008. Such an arrangement may allow for ready interaction between the nitrite providing layer and the activator gel layer to produce nitric oxide, while still absorbing sufficient wound exudate to prevent leakage. In certain embodiments, the activator gel layer 22006 may be a composite ADL such as disclosed herein.

### Perforated Composite ADLs

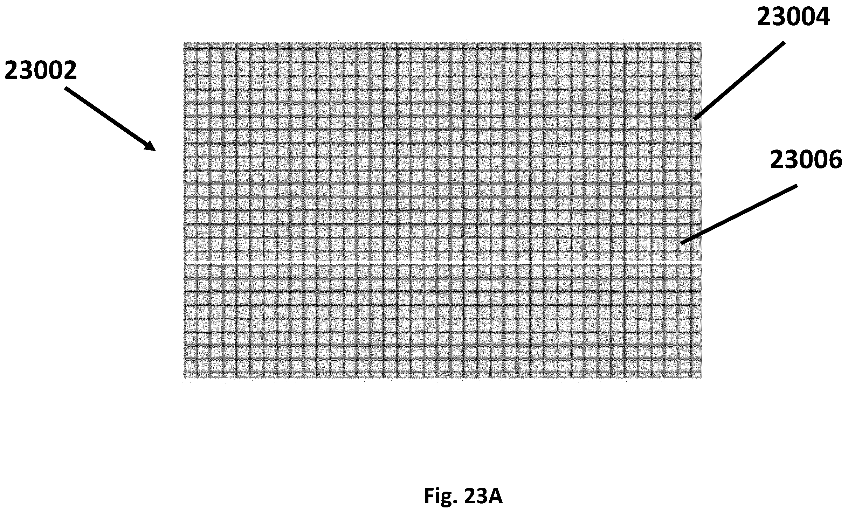

Figures 23A-23E depict wound dressings and composite ADLs with perforations. Such composite ADLs are similar to and may be prepared as described above in relation to Figures 18A through 19B. In some embodiments, the amount of open area in the perforated ADL may range from about: 25-75%, 35% to 65%, 40% to 60%, or about 50%. The perforations may be arranged and shaped to suit the particular embodiment, such as in a pattern. For example, perforations in an ADL may be arranged in lines with the perforations equally spaced and positioned next to each other. In some embodiments, the perforations may be positioned in a checkerboard type pattern with parallel lines and rows of perforations, or perforations maybe offset or staggered relative to each neighboring perforation. The perforations may have any shape outer perimeter, for example, circular, square, rectangular, triangular, diamond, etc. The perforations may have different dimensions as they extend through the material or layer. For example, the perforations may have an overall pyramid shape. Described another way, the perforations may increase or decrease in cross-sectional area as they extend through the layer or material. As will be understood by one of skill in the art, in certain examples, the perforations may be in the form of slits, pits, recesses, bumps, or any suitable conformation.

In embodiments, the ADL within the composite ADL may include prongs, such as microneedles. Such microneedles may penetrate into the activator layer gel to facilitate capillary action into the gel.

Figure 23A depicts a top view of an embodiment of a composite ADL 23002 with perforations in the ADL. Such a composite ADL may include ADL 23004 embedded within activator gel 23006. Perforations through the ADL layer allow the activator gel 23006 to interact directly with nitrite containing fluid passing from a nitrite providing layer, above or below the composite ADL.

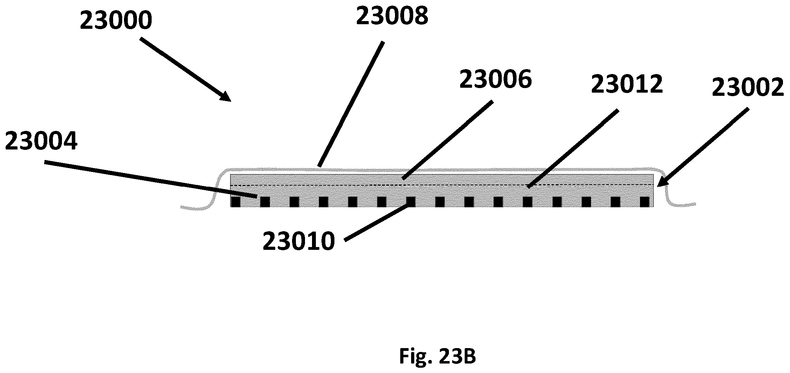

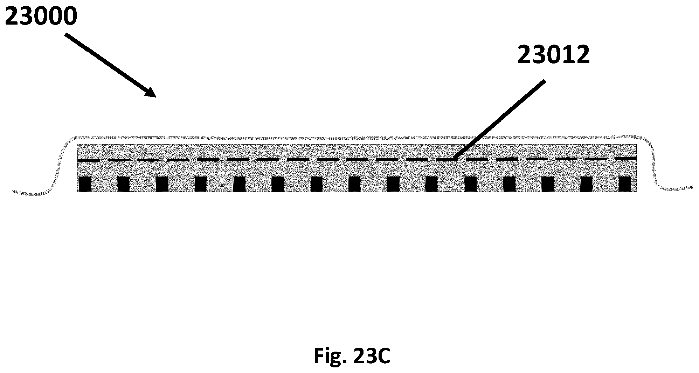

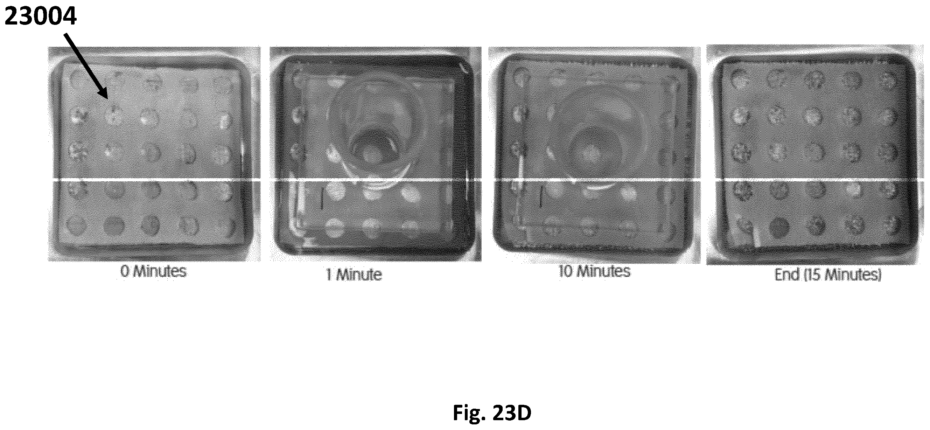

Figure 23B depicts a side view of an embodiment of a dressing 23000 containing a perforated composite ADL 23002, including a perforated ADL 23004 and activator gel 23006 overlain by a cover layer. As described above, the perforated ADL includes perforations 23010, which allow for improved direct fluid flow to the activator gel 23006. In certain examples, the dressing 23000 further includes a mesh interleave 23012. Figure 23C depicts a side view of an embodiment of a dressing 23000 similar to the dressing of Figure 23B. Here, the mesh interleave 23012 includes larger perforations to allow for more fluid flow. In some embodiments, the perforations in the interleave may have an open area of about 25-75%, 35% to 65%, 40% to 60%, or about 50%. In some embodiments, the interleave may be removed entirely to facilitate fluid flow. Figure 23D shows fluid distribution over time from a composite ADL with a perforated ADL layer 23004. As shown in the figures, the fluid is distributed across the composite ADL within a minute after application.

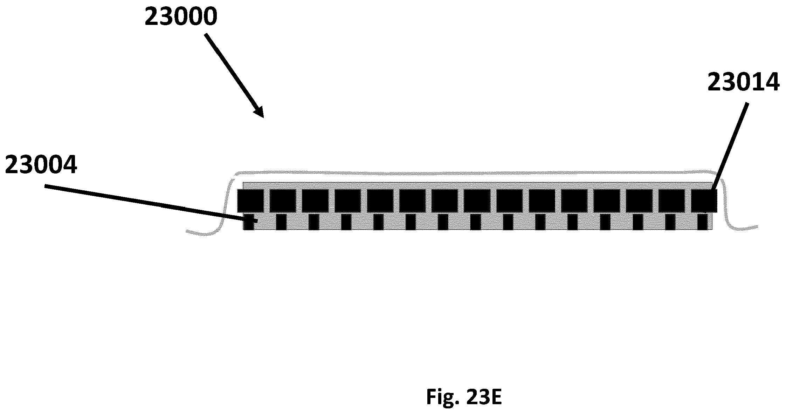

Figure 23E depicts a side view of an embodiment of a wound dressing 23000 similar to the dressings of Figures 23A and 23B. Here, the composite ADL includes a first perforated ADL 23004 and a second overlying perforated ADL 23014. In certain embodiments, the second ADL 23014 may have an open area that is the same as the first ADL 23004, more than the first ADL 23004 or less than the first ADL 23004. In particular embodiments, the ratio of open area in the second ADL 23014 compared to the first ADL 23004 is about: .25, .5, 1, 1.5, 2, 4, 6, or 10.

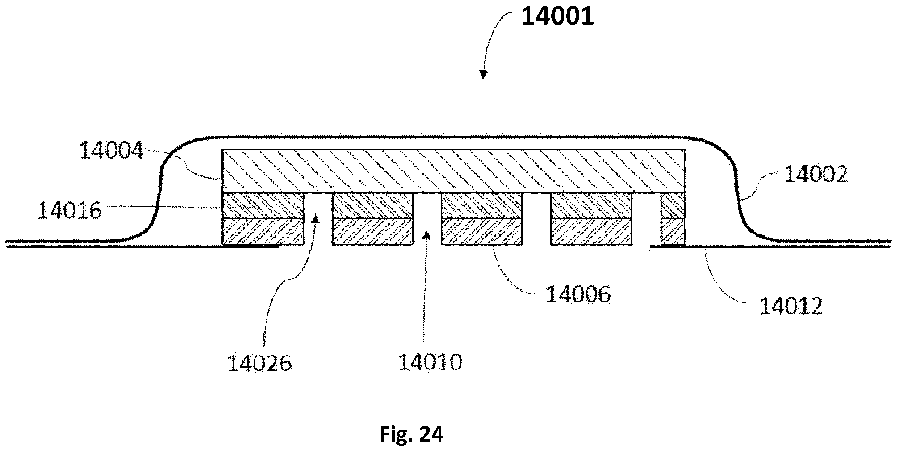

Figure 24 shows another embodiment of a wound dressing 14001, similar to the other wound dressings disclosed herein, such as Figures 18A-23E. The wound dressing 14001 may include the cover layer 14002, the absorbent layer 14004, a gel permeated perforated absorbent layer 14016 such as described elsewhere herein, the gel material or gel layer 14006, and the tissue interface layer 14012. The cover layer 14002 may be placed above the absorbent layer 14004. The gel permeated perforated absorbent layer 14016 may be placed below the absorbent layer 14004. The gel material or gel layer 14006 may be adhered to the wound-facing surface of the gel permeated perforated absorbent layer 14016. The optional tissue interface layer 14012 may be placed below the gel material or gel layer 14006 and adhered to a peripheral portion of the cover layer 14002.

The wound dressing 14001 of Figure 24 may include one or more nitrite providing layers, which also may be separate from the dressing. In some embodiments, the wound dressing 14000 may include an acquisition distribution layer as described herein. The nitrite providing layer(s) such as described herein may be positioned above the absorbent layer 14004, between the absorbent layer 14004 and the gel permeated perforated absorbent layer 14016, between the gel material or gel layer 14006 and the gel permeated perforated absorbent layer 14016, beneath the gel material or gel layer 14006, beneath the cover layer 14002, or between the cover layer 14002 and the absorbent layer 14004. In embodiments having an acquisition distribution layer, the acquisition distribution layer may be placed between the one or more nitrite providing layers and the gel material or gel layer 14006, between the cover layer 14002 and the absorbent layer 14004 or between the absorbent layer 14004 and the gel material or gel layer 14006. As shown in Figure 24, the gel material or gel layer 14006 may have perforations 14010. The perforations may have any suitable arrangement such as described herein, for example in relation to Figures 23A-E

In manufacturing the wound dressing 14001, a gel material may be permeated into a perforated absorbent layer to form the gel permeated perforated absorbent layer 14016. The gel permeated perforated absorbent layer 14016 may then be cured, for example, by using UV light or heat. The gel material or gel layer 14006 may then be adhered to the gel permeated perforated absorbent layer 14016. The size of the perforations 14026 in the gel permeated perforated absorbent layer 14016 may be sized to account for any swelling or expanding caused by permeating the gel material into the perforated absorbent layer.

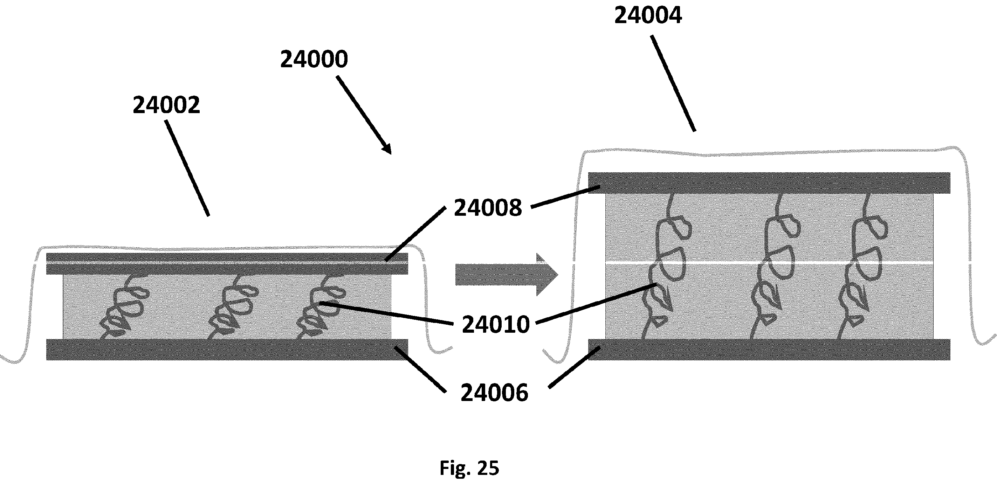

### Embodiments of Figures 25-31

Figure 25 depicts an embodiment of a wound dressing 24000 with a 3D sprung mattress ADL, which may allow the wound dressing to compress 24002 or expand vertically 24004. Such a dressing may include a bottom ADL 24006 and a top ADL 24008, separated by wicking springs 24010. In certain embodiments, the top and bottom ADLs may wick horizontally while the spring elements 24010 wick fluid vertically. As will be understood by one of skill in the art, the dressing may contain a suitable number of wicking springs.

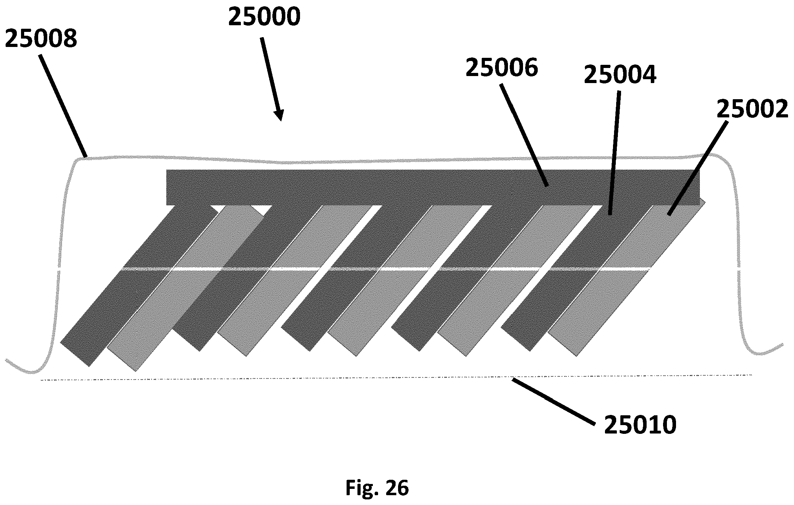

Figure 26 depicts an embodiment of a wound dressing 25000, similar to the wound dressings disclosed above in relation to Figures 18A-18B and elsewhere herein, with alternating layers of activating gel 25002 and ADL 25004, including a top ADL 25006. A cover layer 25008 may overlie the structure and may be adhered to the top ADL 25006 or not adhered. A net layer 25010, which may be a wound contact layer such as disclosed herein, may contain the alternating layers and may be adhered to the cover layer. In certain examples, the layers may not alternate and may instead be randomly ordered or ordered in a suitable arrangement. In certain embodiments, the wound dressing may instead be in the style of a tea bag, with randomly distributed ADL or activating gel contained within a cover layer and net layer. The ratio of ADL to activator gel in the wound dressing 25000 or in the teabag may be about 25:75, 50:50, or 75:25.

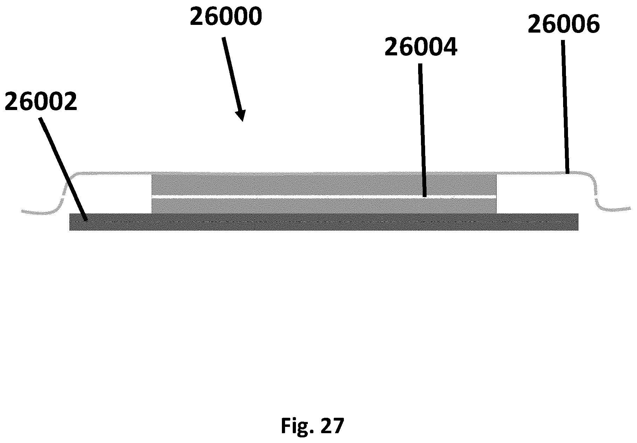

Figure 27 depicts an embodiment of a wound dressing 26000, similar to the wound dressings disclosed above in relation to Figures 18A-18B and elsewhere herein, with an elongated ADL 26002 that extends beyond the borders of the overlying activating gel layer 26004. The elongated ADL may be adhered to the cover layer 26006.

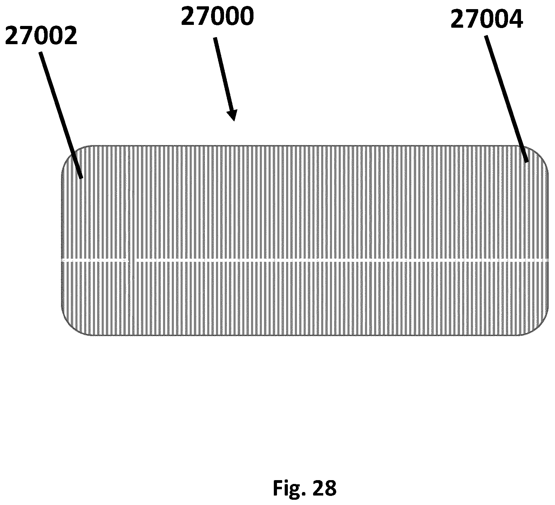

Figure 28 depicts an embodiment of composite layer 27000 with channels of hydrophilic wicking material 27002 that extend vertically through the composite layer, surrounded by activator gel 27004. In certain embodiments, the hydrophilic wicking material may be constructed from any suitable material, such as suitable materials disclosed herein. The hydrophilic wicking material may comprise approximately about: 5%, 10%, 15%, 20%, 25%, 35%, or 50% of the total volume of the composite layer 27000. At least some of the channels 27002 may be oriented in a non-vertical orientation, such as horizontal or at an angle to the vertical axis. In certain embodiments, the channels 27002 may be oriented randomly. In some embodiments, the wicking channels may be oriented toward the central portion of the composite layer 27000, such that fluid is channeled to the center of the composite layer 27000.

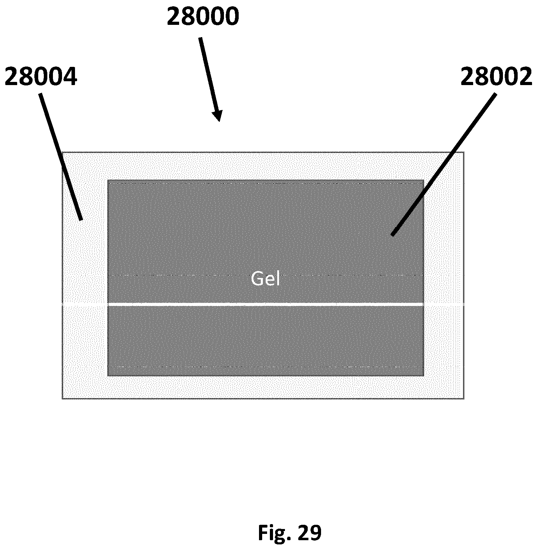

Figure 29 depicts a top view of an embodiment of a dressing 28000, which may include a composite layer 28002 (such as a composite ADL layer, a composite absorbent layer, or any suitable layer). The composite layer 28002 may be surrounded by an absorbent frame 28004 which may serve to act as a barrier to fluid spreading to the peri-wound area.

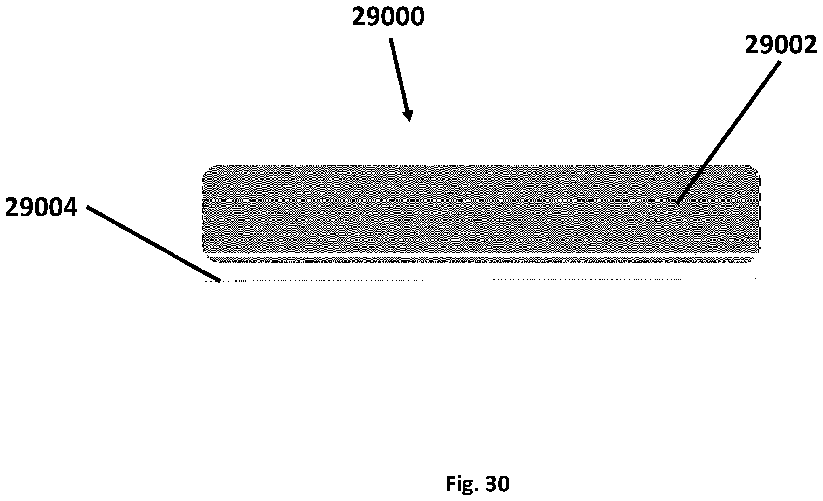

Figure 30 depicts an embodiment of a dressing 29000, which may include a composite layer 29002 (such as a composite ADL layer, a composite absorbent layer, or any suitable layer) and a nitrite providing layer 29004 similar to the other nitrite providing layers 29004 disclosed herein. The nitrite providing layer 29004 may be constructed such that the nitrite providing layer is absorbent, thereby slowing fluid transfer into the composite layer 29002. In certain embodiments the nitrite providing layer may be a mesh with perforations, the perforations may have an open area comprising about 10%, 25%, 50% or 75% of the area of the nitrite providing layer 29004.

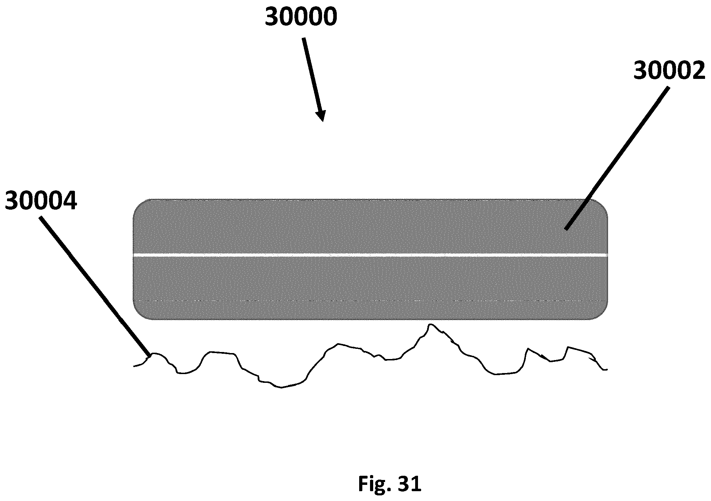

Figure 31 depicts an embodiment of a dressing 30000, which may include a composite layer 30002 (such as a composite ADL layer, a composite absorbent layer, or any suitable layer) and a nitrite providing layer 30004 similar to the other nitrite providing layers 30004 disclosed herein. Here, the nitrite providing layer 30004 may be crumpled, crimped, or roughened so as to increase the surface area for fluid absorption, similar to the dressing of Figure 29. By increasing fluid absorption, the composite layer may absorb wound fluid more slowly and therefore not become over saturated with wound fluid too soon.

### Terminology

Aspects of the disclosure can be modified, if necessary, to employ the systems, functions, and concepts of the various references described herein to provide yet further implementations.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. Furthermore, the features and attributes of the specific embodiments disclosed above may be combined in different ways to form additional embodiments, all of which fall within the scope of the present disclosure.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Likewise, the term "and/or" in reference to a list of two or more items, covers all of the following interpretations of the word: any one of the items in the list, all of the items in the list, and any combination of the items in the list. Further, the term "each," as used herein, in addition to having its ordinary meaning, can mean any subset of a set of elements to which the term "each" is applied. Additionally, the words "herein," "above," "below," and words of similar import, when used in this application, refer to this application as a whole and not to any particular portions of this application.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

Any of the embodiments described herein can be used with a canister or without a canister. Any of the dressing embodiments described herein can absorb and store wound exudate.

## Claims

1. A wound dressing for treating a wound, comprising:
a cover layer configured to form a seal around a wound;
a composite acquisition distribution layer, the composite acquisition distribution layer comprising an activator gel; and
a nitrite source layer, wherein the composite layer is formed from an activator gel cast on an acquisition distribution layer.

2. The wound dressing of Claim 1, wherein the activator gel comprises acidic groups or moieties.

3. The wound dressing of any one of the previous claims, wherein the wound dressing is configured to generate nitric oxide when the wound dressing interacts with fluid.

4. The wound dressing of Claim 3, wherein the fluid comprises wound exudate.

5. The wound dressing of Claim 1, wherein the composite acquisition distribution layer comprises a plurality of perforations.

6. The wound dressing of Claim 5, wherein the perforations comprise an open area of about 25 to 75% of the area of the acquisition distribution layer.

7. The wound dressing of Claim 6, wherein the open area comprises about 50%.

8. A wound dressing for treating a wound, comprising:
a cover layer configured to form a seal around a wound;
a composite absorbent layer, the composite absorbent layer comprising an activator gel; and
a nitrite source layer, wherein the composite absorbent layer further comprises cellulose fibers, gelling fibers, or wherein the activator gel is cast on a foam layer.

9. The wound dressing of Claim 8, wherein the composite absorbent layer comprises ethyl sulphonate fibers

10. The wound dressing of any one of claims 8 and 9, wherein the composite absorbent layer comprises superabsorbent.

11. The wound dressing of Claim 8, wherein the gelling fiber comprises superabsorbent.

12. The wound dressing of any one of Claims 8 to 11, wherein the activator gel is in the form of an array of rounded shapes, or in the form of a plurality of discs embedded in the foam.

## Patentansprüche

1. Wundverband zur Behandlung einer Wunde, umfassend:
eine Deckschicht, gestaltet zum Bilden einer Abdichtung um eine Wunde;
eine Verbund-Aufnahme-Verteilungsschicht, wobei die Verbund-Aufnahme-Verteilungsschicht ein Aktivatorgel umfasst; und
eine Nitritquellenschicht, wobei die Verbundschicht aus einem Aktivatorgel, das auf eine Aufnahme-Verteilungsschicht gegossen ist, gebildet ist.

2. Wundverband nach Anspruch 1, wobei das Aktivatorgel saure Gruppen oder Einheiten umfasst.

3. Wundverband nach einem der vorstehenden Ansprüche, wobei der Wundverband dafür gestaltet ist, Stickoxid zu erzeugen, wenn der Wundverband mit Fluid wechselwirkt.

4. Wundverband nach Anspruch 3, wobei das Fluid Wundexsudat umfasst.

5. Wundverband nach Anspruch 1, wobei die Verbund-Aufnahme-Verteilungsschicht eine Vielzahl von Perforationen umfasst.

6. Wundverband nach Anspruch 5, wobei die Perforationen eine offene Fläche von etwa 25 bis 75 % der Fläche der Aufnahme-Verteilungsschicht umfassen.

7. Wundverband nach Anspruch 6, wobei die offene Fläche etwa 50 % umfasst.

8. Wundverband zur Behandlung einer Wunde, umfassend:
eine Deckschicht, gestaltet zum Bilden einer Abdichtung um eine Wunde;
eine absorbierende Verbundschicht, wobei die absorbierende Verbundschicht ein Aktivatorgel umfasst; und
eine Nitritquellenschicht, wobei die absorbierende Verbundschicht ferner Cellulosefasern, gelbildende Fasern umfasst oder wobei das Aktivatorgel auf eine Schaumstoffschicht gegossen ist.

9. Wundverband nach Anspruch 8, wobei die absorbierende Verbundschicht Ethylsulfonatfasern umfasst.

10. Wundverband nach einem der Ansprüche 8 bis 9, wobei die absorbierende Verbundschicht Superabsorbens umfasst.

11. Wundverband nach Anspruch 8, wobei die gelbildende Faser Superabsorbens umfasst.

12. Wundverband nach einem der Ansprüche 8 bis 11, wobei das Aktivatorgel in der Form einer Anordnung von abgerundeten Formen oder in Form einer Vielzahl von in den Schaumstoff eingebetteten Scheiben vorliegt.

## Revendications

1. Pansement destiné au traitement d'une plaie, comprenant :
une couche de revêtement conçue pour former un joint d'étanchéité autour d'une plaie ;
une couche de répartition d'acquisition composite, la couche de répartition d'acquisition composite comprenant un gel activateur ; et
une couche source de nitrite, la couche composite étant formée à partir d'un gel activateur versé sur une couche de répartition d'acquisition.

2. Pansement de la revendication 1, dans lequel le gel activateur comprend des groupes ou fractions acides.

3. Pansement de l'une quelconque des revendications précédentes, le pansement étant conçu pour générer de l'oxyde nitrique lorsque le pansement interagit avec un fluide.

4. Pansement de la revendication 3, le fluide comprenant un exsudat de plaie.

5. Pansement de la revendication 1, dans lequel la couche de répartition d'acquisition composite comprend une pluralité de perforations.

6. Pansement de la revendication 5, dans lequel les perforations présentent une superficie ouverte représentant environ 25 à 75 % de la superficie de la couche de répartition d'acquisition.

7. Pansement de la revendication 6, dans lequel la superficie ouverte représente environ 50 %.

8. Pansement destiné au traitement d'une plaie, comprenant :
une couche de revêtement conçue pour former un joint d'étanchéité autour d'une plaie ;
une couche absorbante composite, la couche absorbante composite comprenant un gel activateur ; et
une couche source de nitrite, la couche absorbante composite comprenant en outre des fibres de cellulose, des fibres gélifiantes, ou bien le gel activateur étant versé sur une couche de mousse.

9. Pansement de la revendication 8, dans lequel la couche absorbante composite comprend des fibres de sulfonate d'éthyle.

10. Pansement de l'une quelconque des revendications 8 et 9, dans lequel la couche absorbante composite comprend un superabsorbant.

11. Pansement de la revendication 8, dans lequel la fibre gélifiante comprend un superabsorbant.

12. Pansement de l'une quelconque des revendications 8 à 11, dans lequel le gel activateur se présente sous la forme d'un réseau de formes arrondies, ou sous la forme d'une pluralité de disques incorporés dans la mousse.
